# FASCICULE DE BREVET EUROPEEN

(11) **EP 1 200 052 B1**
(45) Date de publication et mention de la délivrance du brevet: **26.05.2010**
(21) Numéro de dépôt: 01913996.3
(22) Date de dépôt: 13.03.2001
(51) Int. Cl.: A61Q 5/06, A61K 8/49

(54) **COMPOSITIONS POUR LA TEINTURE DES FIBRES KERATINIQUES CONTENANT DES DERIVES DE PARAPHENYLENEDIAMINE A GROUPEMENT PYRROLIDINYLE**
ZUSAMMENSETZUNG ZUR FÄRBUNG VON KERATINISCHEN FASERN ENTHALTEND PARAPHENYLENDIAMIN-DERIVATE MIT EINER PYRROLIDINYL-GRUPPE
DYEING COMPOSITIONS FOR KERATINOUS FIBRES CONTAINING PARAPHENYLENEDIAMINE DERIVATIVES WITH PYRROLIDINYL GROUP

(30) Priorité: 14.03.2000 FR 0003250
(43) Date de publication de la demande: 02.05.2002
(73) Titulaire: L'Oréal, 75008 Paris (FR)
(72) Inventeur: VIDAL, Laurent, F-75013 Paris (FR); TERRANOVA, Eric, F-06520 Magagnosc (FR); SABELLE, Stéphane, F-75005 Paris (FR)
(74) Mandataire: Miszputen, Laurent
(86) Numéro de dépôt international: PCT/FR2001/000745
(87) Numéro de publication internationale: WO 2001/068043

(56) Documents cités:
- EP-A- 0 962 452
- DE-A- 4 241 532
- US-A- 5 851 237
- US-A- 5 876 464
- PATENT ABSTRACTS OF JAPAN vol. 1999, no. 11, 30 septembre 1999 (1999-09-30) & JP 11 158048 A (FUJI PHOTO FILM CO LTD), 15 juin 1999 (1999-06-15) cité dans la demande & JP 11 158048 A 15 juin 1999 (1999-06-15) cité dans la demande

## Description

L'invention a pour objet de nouvelles compositions pour la teinture d'oxydation des fibres kératiniques comprenant au moins un dérivé de paraphénylènediamine à groupement pyrrolidinyle à titre de base d'oxydation, un procédé de teinture et un kit de teinture mettant en oeuvre cette composition.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des composés hétérocycliques tels que des dérivés de diaminopyrazole, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Dans le domaine de la coloration capillaire, la paraphénylènediamine et la paratoluylènediamine sont des bases d'oxydation largement utilisées. Elles permettent d'obtenir avec des coupleurs d'oxydation des nuances variées.

Cependant, il existe un besoin de découvrir de nouvelles bases d'oxydation, encore appelées développeurs, présentant un meilleur profil toxicologique que la paraphénylènediamine ou la paratoluylènediamine, tout en permettant de conférer aux cheveux d'excellentes propriétés d'intensité de couleur, de variété de nuances, d'uniformité de la couleur et de ténacité vis à vis des agents extérieurs.

Il a déjà été proposé, notamment dans la demande de brevet GB 2 239 265, d'utiliser de la 2-(β-hydroxyéthyl) paraphénylènediamine ou bien encore de la N,N-bis-(β-hydroxyéthyl) paraphénylènediamine comme des remplaçants potentiels de la paraphénylènediamine et de paratoluylènediamine. Il en est de même pour les 2-(hydroxyalcoxy) paraphénylènediamines, (voir notamment le brevet US 5 538 516).

Cependant, la N,N-bis-(β-hydroxyéthyl) paraphénylènediamine et la 2-(β-hydroxyéthyl paraphénylènediamine présentent néanmoins l'inconvénient de conduire à une variété de nuances plus limitée et de conférer moins d'intensité de couleur, moins d'uniformité aux cheveux que la paraphénylènediamine ou la paratoluylènediamine. Il en est de même pour les 2-(hydroxyalcoxy) paraphénylènediamines qui confèrent aux cheveux une couleur qui évolue et change au cours du temps.

Par ailleurs, il est connu dans la littérature, voir R.L. Bent et coll. , J.A.C.S. 73, 3100, 1951, que les dérivés de paraphénylènediamine dont un des atomes d'azote est compris dans un cycle non aromatique à 6 ou 7 chaînons carbonés ou hétérocycliques sont moins oxydables que les dérivés de paraphénylènediamine dont un des atomes d'azote est substitué par deux substituants dissymétriques, qui sont eux-mêmes moins oxydables que les dérivés de paraphénylènediamine dont un des atomes d'azote est substitué par deux substituants symétriques.

Il est également mentionné dans le même article que le dérivé de paraphénylènediamine dont un des atomes d'azote est compris dans un cycle non aromatique à 5 chaînons carboné est plus oxydable que chacun des dérivés cités ci dessus. Cette classe particulière de dérivés N-pyrrolidinique de paraphénylènediamine permet donc d'obtenir des réactions de condensation cinétiquement accélérées avec des coupleurs en milieu basique et oxydant par rapport aux dérivés de paraphénylènediamine cités ci-dessus.

Cependant, les bases d'oxydation qui sont trop oxydables et qui réagissent avec des coupleurs selon des vitesses de réaction accélérées, conduisent généralement à la formation des colorants à l'extérieur de la fibre kératinique. Les intensités, les ténacités et l'uniformité des colorations ainsi obtenues sur les cheveux sont généralement insuffisantes.

Toutefois, le brevet US 5,851,237 propose l'utilisation de dérivés 1-(4-aminophényl)pyrrolidine éventuellement substitués sur le noyau benzénique afin de remplacer la paraphénylènediamine. A ce titre, le même brevet propose très préférentiellement l'utilisation de la 1-(4-aminophényl)pyrrolidine comme substitut de la paraphénylènediamine.

Or, il est connu dans la littérature que la 1-(4-aminophényl)pyrrolidine possède une activité fortement allergène, (R.L. Bent et coll., J.A.C.S. 73, 3100, 1951).

Le brevet US 5,993,491 propose également l'utilisation de dérivés N-(4-aminophényl)-2-(hydroxyméthyl)-pyrrolidines éventuellement substituées sur le noyau benzénique afin de remplacer la paraphénylènediamine. Comme composés revendiqués très préférentiels, ledit brevet propose la N-(4-aminophényl)-2-(hydroxyméthyl)-pyrrolidine éventuellement substituée par un radical méthyle en position 3.

Cependant, il a clairement été établi que ces composés ne permettent pas de conférer aux cheveux une coloration de qualité équivalente à celle obtenue avec la paraphénylènediamine, du fait d'un manque d'intensité et d'uniformité de la couleur obtenue.

La demande de brevet JP 11158048 propose également des compositions de coloration capillaire offrant de bonnes propriétés d'étalement, de facilité d'application et de tenue au shampooing. Ces compositions tinctoriales contiennent au moins un composé choisi parmi des dérivés de paraphénylènediamine éventuellement substitués par 1 à 4 substituants sur le noyau benzénique et dont un des atomes d'azote est compris dans un cycle de 5 à 7 chaînons carboné ou parmi des dérivés de paraphénylènediamine éventuellement substitués par 1 à 4 substituants sur le noyau benzénique et dont un des atomes d'azote est substitué par un radical Z₁ et un radical Z₂, Z₁ étant un groupe alkyle, aryle ou un hétérocycle, et Z₂ étant un radical -(CH₂-CH₂-O)-Z₃ dans lequel Z₃ représente un atome d'hydrogène, un groupe alkyle, aryle ou un hétérocycle.

En terme de pouvoir colorant, de facilité d'application, d'uniformité de la coloration obtenue et de ténacité notamment vis à vis de l'action des shampoings, Il apparaît dans cette demande de brevet japonais que les dérivés comportant un cycle à 5 chainons, à savoir la N-(3-isopropyloxy-4-aminophényl)-2,5-di-éthyl pyrrolidine, la N-(3-méthyl-4-aminophényl)-3-(2-hydroxyéthyloxy) pyrrolidine et la N-(3-méthyl-4-aminophényl)-2-méthyl-4-hydroxypyrrolidine, se comportent comme des bases d'oxydation dérivés de 4-aminoaniline dont l'atome d'azote est compris dans un cycle à 6 chaînons pipéridinique fonctionnaalisé.

Cependant, il est Connu que lorsque l'un des atomes d'azote des dérivés de paraphénylènediamine est compris dans un cycle à 6 chaînons, l'énergie d'activation pour conduire à la forme oxydée quinone-imine correspondante est parmi des plus élevée en série paraphénylènediamine N,N-disubstituées. Cette donnée a pour conséquente que les réactions de condensation avec des coupleurs sont moins efficaces et confèrent aux cheveux des propriétés de coloration insuffisante en terme d'intensité et d'uniformité de la couleur obtenue, comparativement à celles obtenues avec la paraphénylènediamine ou la paratoluylènediamine.

Il en résulte que les solutions proposées dans la demande de brevet JP 11158048, au travers des dérivés de paraphénylènediamine ayant un atome d'azote compris dans un cycle pyrrolidinique fonctionnalisé, ne permettent pas de conférer des résultats tinctoriaux sur cheveux, équivalents à ceux obtenus avec la paraphénylènediamine ou la paratoluylènediamine.

Il est donc clair qu'il existe un réel besoin de découvrir de nouvelles bases d'oxydation présentant à la fois un bon profil toxicologique et des propriétés telles que les compositions les contenant permettent de conférer aux cheveux des colorations présentant d'excellentes propriétés d'intensité de couleur, de variété de nuances, d'uniformité de la couleur (e'est à dire une faible sélectivité), et de ténacité vis à vis des différentes agressions extérieures que peuvent subir les cheveux.

Or, la demanderesse vient maintenant de découvrir, de façon totalement inattendue et surprenante, que certains dérivés de paraphénylènediamine à groupement pyrrolidinyle de formule (I) définie ci-après, non seulement conviennent pour une utilisation à titre de base d'oxydation pour la teinture d'oxydation des fibres kératiniques, mais en outre qu'ils conduisent à des colorations particulièrement puissantes et peu sélectives. Ils permettent de plus d'obtenir des compositions tinctoriales conduisant à des colorations résistant bien aux diverses agressions que peuvent subir les cheveux.

Ces découvertes sont à la base de la présente invention.

L'invention a donc pour premier objet une composition pour la teinture d'oxydation des fibres kératiniques et en particulier des fibres kératiniques humaines telles que les cheveux, caractérisée par le fait qu'elle comprend, dans un milieu approprié pour la teinture, au moins une base d'oxydation choisie parmi les dérivés de paraphénylènediamine à groupement pyrrolidinyle la N-(4-aminophenyl)-3-hydoxypyrrolidine, la N-(4-amino3-methylphenyl)-3-hydoxypyrrolidine, la N-(4-amino 2- methylphenyl)-3-hydoxypyrrolidine, la N-(4-amino 2-ethylphenyl)-3-hydoxypyrrolidine, la N-3-ethylphenyl) 3-hydroxypyrrolidine, et leurs sels d'addition avec un acide,

Elles comprennent en outre un ou plusieurs coupleurs choisis parmi les métaphénylènediamines, les méta-aminophénols et les coupleurs hétérocycliques.

Comme indiqué précédemment, les colorations obtenues avec la composition de teinture d'oxydation conforme à l'invention sont puissantes et peu sélectives, et présentent en outre d'excellentes propriétés de résistance vis à vis de l'action des différents agents extérieurs (lumière, intempéries, lavage, ondulation permanente, transpiration, frottements). Les compositions de teinture d'oxydation conformes à l'invention permettent de plus d'atteindre des nuances dans une très large palette de couleurs.

Les dérivés de paraphénylènediamine à groupement pyrrolidinyle selon l'invention, ainsi que leurs procédés de synthèse sont connus, voir notamment la demande de brevet DE 4 241 532(AGFA).

Le ou les dérivés de paraphénylènediamines à groupement pyrrolidinyle conforme à l'invention et/ou le ou leurs sels d'addition avec un acide représentent de préférence de 0,0005 à 12% en poids environ du poids total de la composition tinctoriale, et encore plus préférentiellement de 0,005 à 6 % en poids environ de ce poids.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre I et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Selon un mode de réalisation particulier, les agents alcalinisants sont choisis parmi les alcanolamines, les diaminoalcanes ou l'ammoniaque.

Au sens de la présente invention, on entend par alcanolamine, tout composé hydrocarboné comportant de 2 à 100 atomes de carbone, saturé ou insaturé, linéaire ou ramifié et comportant (i) au moins une fonction amine éventuellement substituée par un ou deux substituants qui sont de préférence alkyle en C₁-C₄ ou alkyle en C₁-C₄ substitué comme par exemple mono- out poly-hydroxyalkyle, et (ii) au moins une fonction hydroxyle non portée par la fonction amine. De préférence la chaîne hydrocarbonée est saturée.

Parmi les alcanolamines utilisables selon l'invention, on peut citer la monoéthanolamine, la diéthanolamine, la triéthanolamine, la triisopropanolamine, le 2-amino-2-méthyl-1-propanol, le 2-amino-2-méthyl-1,3-propanediol, le 2-amino-2-éthyl-1,3-propanediol, le 2-amino-1-butanol, le tris-(hydroxyméthyl)-aminométhane, la 2-aminoéthanolamine, le 1-diéthylamino-2,3-propanediol, le 2-diméthylamino-2-méthyl-1-propanol, la diméthyléthanolamine, la diéthyléthanolamine, l'éthylmonoéthanolamine, la méthyléthanolamine. De préférence, on utilise la monoéthanolamine.

Les diaminoalcanes utilisés selon l'invention sont de préférence les diaminoalcanes de formule (II) suivante : 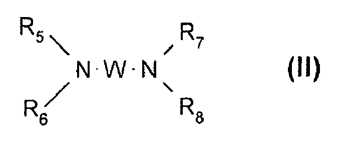 dans laquelle, W est un reste alkylène éventuellement substitué par un groupement hydroxyle ou un radical alkyle en C₁-C₄ ; R₅, R₆, R₇ et R₈, identiques ou différents, représentent un atome d'hydrogène, un radical alkyle en C₁-C₄ ou hydroxyalkyle en C₁-C₄. De préférence W est un reste propylène. Encore plus préférentiellement le diaminoalcane est le diamino-propane.

Parmi tous les agents alcalins utilisables selon l'invention, on utilise de préférence l'ammoniaque.

Le ou les agents alcalins sont présents dans la composition tinctoriale d'oxydation prête à l'emploi ou la composition destinée à la teinture d'oxydation selon l'invention dans des concentrations allant d'environ 0,1 à environ 20% en poids, et de préférence d'environ 0,5 à environ 10% en poids de matières actives par rapport au poids total de la composition destinée à la teinture d'oxydation ou la composition tinctoriale d'oxydation prête à l'emploi.

Selon une forme de réalisation préférée, la composition de teinture d'oxydation conforme à l'invention renferme en outre un ou plusieurs coupleurs de façon à modifier ou à enrichir en reflets les nuances obtenues en mettant con oeuvre les composés de formule (1).

Les coupleurs utilisables dans les compositions de teinture d'oxydation conformes à l'invention sont choisis parmi les métaphénylènediamines, les méta-aminophénols, les métadiphénols et les coupleurs hétérocycliques, par exemple les pyrazolo-[1,5-b]-1,2,4-triazoles, les pyrazolo-[3,2-c]-1,2,4-triazoles, les pyrazol-5-ones, les pyridines, les indoles, les indolines, les indazoles, les benzimidazoles, les benzothiazoles, les benzoxazoles, les 1,3-benzodioxoles et les quinolines.

Selon un mode de réalisation particulier, le ou les coupleurs sont choisis parmi les coupleurs hétérocycliques, les métadiphénols substitués, les métaphénylènediamines substituées, les naphtols et naphtols acylés, et les métaaminophénols de formule (III) suivante, et leurs sels d'addition avec un acide : 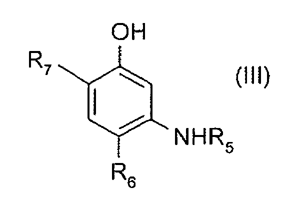 dans laquelle :
- R₅ représente un atome d'hydrogène, un radical alkyle en C₁-C₄, monohydroxyalkyle en C₁-C₄, ou polyhydroxyalkyle en C₂-C₄,
- R₆ représente un atome d'hydrogène, un radical alkyle en C₁-C₄, alcoxy en C₁-C₄, ou un atome d'halogène choisi parmi le chlore, le brome ou le fluor,
- R₇ représente un atome d'hydrogène, un radical alkyle en C₁-C₄, alcoxy en C₁-C₄, monohydroxyalkyle en C₁-C₄, polyhydroxyalkyle en C₂-C₄, monohydroxyalcoxy en C₁-C₄, ou polyhydroxyalcoxy en C₂-C₄

Parmi les méta-diphénols substitués utilisables à titre de coupleurs dans la composition tinctoriale conforme à l'invention, on utilisera de préférence les composés de formule (VI) suivante, et leurs sels d'addition avec un acide : 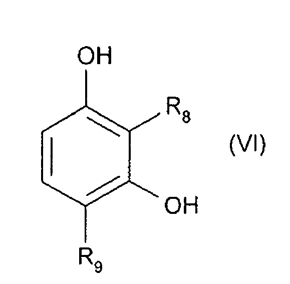 dans laquelle :
R₈ et R₉, identiques ou différents, représentent un atome d'hydrogène, un radical alkyle en C₁-C₄, ou un atome d'halogène choisi parmi le chlore, le brome ou le fluor, étant entendu qu'au moins un des radicaux R₈ et R₉ est différent d'un atome d'hydrogène.

Parmi les méta-diphénols substitués de formule (VI) ci-dessus, on peut plus particulièrement citer le 2-méthyl-1,3-dihydroxy benzène, le 4-chloro-1,3-dihydroxy benzène, le 2-chloro-1,3-dihydroxybenzène, et leurs sels d'addition avec un acide.

Parmi les métaphénylènediamines substituées utilisables à titre de coupleurs dans la composition tinctoriale conforme à l'invention, on utilisera de préférence les composés de formule (V) suivante, et leurs sels d'addition avec un acide : 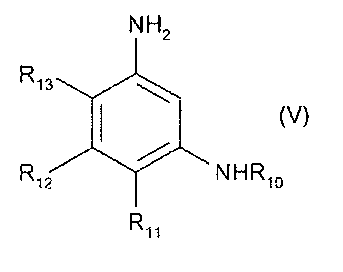 dans laquelle :
- R₁₀ représente un atome d'hydrogène, un radical alkyle en C₁-C₄, monohydroxyalkyle en C₁-C₄, ou polyhydroxyalkyle en C₂-C₄ ;
- R₁₁ et R₁₂, identiques ou différents, représentent un atome d'hydrogène, un radical alkyle en C₁-C₄, monohydroxyalcoxy en C₁-C₄, ou polyhydroxyalcoxy en C₂-C₄ ;
- R₁₃ représente un atome d'hydrogène, un radical alkoxy en C₁-C₄, aminoalkoxy en C₁-C₄, monohydroxyalkoxy en C₁-C₄, polyhydroxyalkoxy en C₂-C₄, ou un radical 2,4-diaminophénoxyalkoxy ; étant entendu qu'au moins un des radicaux R₁₀ à R₁₃ est différent d'un atome d'hydrogène.

Parmi les méta-phénylènediamines substituées de formule (V) ci-dessus, on peut plus particulièrement citer le 3,5-diamino-1-éthyl-2-méthoxybenzène, le 3,5-diamino 2-méthoxy-1-méthyl-benzène, le 2,4-diamino-1-éthoxybenzène, le 1,3-bis-(2,4-diaminophénoxy) propane, le bis-(2,4-diaminophénoxy) méthane, le 1-(β-aminoéthyloxy) 2,4-diamino benzène, le 2-amino 1-(β-hydroxyéthyloxy) 4-méthylamino benzène, le 2,4-diamino-1-éthoxy-5-méthyl-benzène, le 2,4-diamino-5-(β-hydroxyéthyloxy) 1-méthylbenzène, le 2,4-diamino 1-(β,γ-dihydroxypropyloxy) benzène, le 2,4-diamino 1-(β-hydroxyéthyloxy) benzène, le 2-amino 4-N-(β-hydroxyéthyl) amino 1-méthoxy benzène, et leurs sels d'addition avec un acide.

Parmi les coupleurs hétérocycliques utilisables dans la composition tinctoriale conforme à l'invention, on peut notamment citer les dérivés indoliques, les dérivés indoliniques, les dérivés de benzimidazole, les dérivés de benzomorpholine, les dérivés de sésamol, les dérivés pyrazolo-azoliques, les dérivés pyrrolo-azoliques, les dérivés imidazolo-azoliques, les dérivés pyrazolo-pyrimidiniques, les dérivés de pyrazolin-3,5-diones, les dérivés pyrrolo-[3,2-d]-oxazoliques, les dérivés pyrazolo-[3,4-d]-thiazoliques, les dérivés S-oxyde-thiazolo-azoliques, les dérivés S,S-dioxyde-thiazolo-azoliques, et leurs sels d'addition avec un acide.

Parmi les dérivés indoliques utilisables à titre de coupleurs hétérocycliques dans la composition tinctoriale conforme à l'invention, on peut particulièrement citer les composés de formule (VI) suivante, et leurs sels d'addition avec un acide : 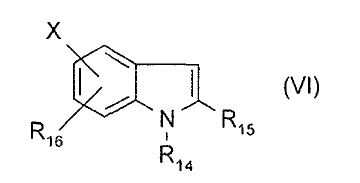 dans laquelle :
- R₁₄ représente un atome d'hydrogène, un radical allyle en C₁-C₄, monohydroxyalkyle en C₁-C₄, polyhydroxyalkyle en C₂-C₄, ou aminoalkyle en C₁-C₄ dont l'amine est mono ou disubstituée par un groupement alkyle en C₁-C₄ ;
- R₁₅ représente un atome d'hydrogène ou un radical alkyle en C₁-C₄ ;
- R₁₆ représente un atome d'hydrogène, un radical alkyle en C₁-C₄ ou hydroxyle ;
- X représente un radical hydroxyde ou NHR₁₇ dans lequel R₁₇ représente un atome d'hydrogène, un radical alkyle en C₁-C₄, ou hydroxyalkyle en C₁-C₄.

Parmi les dérivés indoliques de formule (V) ci-dessus, on peut plus particulièrement citer le 4-hydroxy indole, le 6-hydroxy indole, le 7-amino idole, le 6-amino indole, le 7-hydroxy indole, le 7-éthyl 6-(β-hydroxyéthyl)amino indole, le 4-amine indole, le 6-hydroxy 1-méthyl indole, le 5,6-dihydroxy indole, le 4-hydroxy 1-N-méthyl indole, le 4-hydroxy 2-méthyl indole, le 4-hydroxy 5-méthyl indole, le 4-hydroxy 1-N-(β-hydroxyéthyl) indole, le 4-hydroxy 1-N-(β-hydroxypropyl) indole, le 1-N-(β,γ-dihydroxypropyl) 4-hydroxy indole, le 4-hydroxy 1-N-(β-hydroxyéthyl) 5-méthyl indole, le 1-N-(γ-diméthylaininopropyl) 4-hydroxy indole, et leur sels d'addition avec un acide.

Parmi les dérivés indoliniques utilisables à titre de coupleurs hétérocycliques dans la composition tinctoriale conforme à l'invention, on peut particulièrement citer la 4-hydroxy indoline, la 6-hydroxy indoline, la 6-amino indoline, la 5,6-dihydroxy indoline, et leurs sels d'addition avec un acide.

Parmi les dérivés de benzimidazole utilisables à titre de coupleurs hétérocycliques dans la composition tinctoriale conforme à l'invention, on peut plus particulièrement eiter les composés de formule (VII) suivante, et leurs sels d'addition avec un acide : 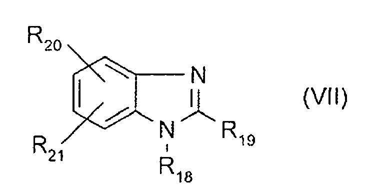 dans laquelle :
- R₁₈ représente un atome d'hydrogène ou un radical alkyle en C₁-C₄,
- R₁₉ représente un atome d'hydrogène, une radical alkyle en C₁-C₄, ou phényle,
- R₂₀ représente un radical hydroxyle, amino ou méthoxy,
- R₂₁ représente un atome d'hydrogène, un radical hydroxyde, méthoxy ou alkyle en C₁-C₄ sous réserve que :
   - lorsque R₂₀ désigne un radical amino, alors il occupe la position 4,
   - lorsque. R₂₀ occupe la position 4, alors R₂₁, occupe la position 7,
   - lorsque R₂₀ occupe la position 5, alors R₂₁ occupe la position 6.

Parmi les dérivés de benzimidazole de formule (VII) ci-dessus, on peut plus particulièrement citer le 4-hydroxy benzimidazole, le 4-amine benzimidazole, le 4-hydroxy 7-tnéthyl benzimidazole, le 4-hydroxy 2-métltyl benzimidazole, le 1-butyl 4-hydroxy benzimidazole, le 4-amino 2-méthyl benzimidazole, le 5,6-dihydroxy benzimidazole, le 5-hydroxy 6-méthoxy benzimidazole, le 4,7-dihydroxy benzimidazole, le 4,7-dihydroxy 1-méthyt benzimidazole, le 4,7-diméthoxy benzimidazole, le 5,6-dihydroxy 1-méthyl benzimidazole, le 5,6-dihydroxy 2-méthyl benzimidazole, le 5,6-diméthoxy benzimidazole, et leurs sels d'addition avec un acide.

Parmi les dérivés de benzomorpholine utilisables à titre de coupleurs hétérocycliques dans la composition tinctoriale conforme à l'invention, on peut plus particulièrement citer les composés de formule (VIII) suivante, et leurs sels d'addition avec un acide : 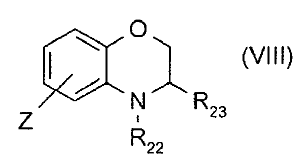 dans laquelle:
R₂₂ et R₂₃, identiques ou différents, représentent un atome d'hydrogène ou un radical alkyle en C₁-C₄,
Z représente un radical hydroxyle ou amino.

Parmi les dérivés de benzomorpholine de formule (VIII) ci-dessus, on peut pus particulièrement citer la 6-hydroxy 1,4-benzomorpholine, la N-méthyl 6-hydroxy 1,4-benzomorpholine, la 6-amino 1,4-benzomorpholine, et leurs sels d'addition avec un acide.

Parmi les dérivés de sésamol utilisables à titre de coupleurs hétérocycliques dans la composition tinctoriale conforme à l'invention, on peut particulièrement citer les composés de formule (IX) suivante, et leurs sels d'addition avec un acide : 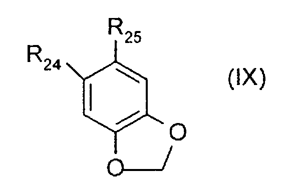 dans laquelle :
R₂₄ désigne un radical hydroxyle, amino, alkyl(C₁-C₄)amino, monohydroxyalkyl(C₁-C₄)amino ou polyhydroxyalkyl(C₂-C₄)amino,
R₂₅ désigne un atome d'hydrogène ou d'halogène ou un radical alcoxy en C₁-C₄.

Parmi les dérivés de sésamol de formule (IX) ci-dessus, on peut plus particulièrement citer le 2-bromo 4,5-méthylènedioxy phénol, la 2-méthoxy 4,5-méthylènedioxy aniline, le 2-(β-hydroxyéthyl)amino 4,5-méthylènedioxy benzène, et leurs sels d'addition avec un acide.

Parmi les dérivés pyrazolo-azoliques utilisables à titre de coupleurs hétérocycliques dans la composition tinctoriale conforme à l'invention, on peut plus particulièrement citer les composés décrits dans les demandes de brevets et brevets suivants : FR-A-2 075 583, EP-A-0 119 860, EP-A-0 285 274, EP-A-0 244 160, EP-A-0 578 248, GB 1 458 377, US 3 227 554, US 3 419 391, US 3 061 432, US 4 500 630, US 3 725 067, US 3 926 631, US 5 457 210, JP 84/99437, JP 83/42045, JP 84/162548, JP 84/171956, JP 85/33552, JP 85/43659, JP 85/172982, et JP 85/190779, ainsi que dans les publications suivantes : Chem. Ber. 32, 797 (1899), Chem. Ber. 89, 2550, (1956), J. Chem. Soc. Perkin trans 1, 2047, (1977), J. Prakt. Chem., 320, 533, (1978) ; dont les enseignements font partie intégrante de la présente demande.

A titre de dérivés pyrazolo-azoliques, on peut tout particulièrement citer :
- le 2-méthyl pyrazolo [1,5-b]-1,2,4-triazole,
- le 2-éthyl pyrazolo [1,5-b]-1,2,4-triacole,
- le 2-isopropyl pyrazolo [1,5-b]-1,2,4-triazole,
- le 2-phényl pyrazolo [1,5-b]-1,2,4-triazole,
- le 2,6-diméthyl pyrazolo [1,5-b]- 1,2,4-triazole,
- le 7-chloro-2,6-diméthylpyrazolo[1,5-b]-1,2,4-triarole,
- le 3,6-diméthyl-pyrazolo [3,2-c]-1,2,4-triazole,
- le 6-phényl-3-méthylthio- pyrazolo [3,2-c]-1,2,4-triazole,
- le 6-amino- pyrazolo [1,5-a] benzimidazole,
et leurs sels d'addition avec un acide.

Parmi les dérivés pyrrolo-azoliques utilisables à titre de coupleurs hétérocycliques dans la composition tinctoriale conforme à l'invention, on peut plus particulièrement citer les composés décrits dans les demandes de brevets et brevets suivants : US 5 256 526, EP-A-0 557 851, EP-A-0 578 248, EP-A-0 518 238, EP-A-0 456 226, EP-A-0 488 909, EP-A-0 488 248, et dans les publications suivantes :
- D.R. Liljegren Ber. 1964, 3436 ;
- E.J. Browne, J.C.S., 1962, 5149 ;
- P. Magnus, J.A.C.S., 1990, 112, 2465 ;
- P. Magnus, J.A.C.S., 1987, 109, 2711 ;
- Angew. Chem. 1960, 72, 956 ; et
- Rec. Trav. Chim. 1961, 80, 1075 ; dont les enseignements font partie intégrante de la présente demande.

A titre de dérivés pyrrolo-azoliques, on peut tout particulièrement citer :
- le 5-cyano-4-éthoxycarbonyl-8-méthyl pyrrolo [1,2-b]-1,2,4-triazole,
- le 5-cyano-8-méthyl-4-phényl pyrrolo [1,2-b]-1,2,4-triazole,
- le 7-amido-6-éthoxycarbonyl pyrrolo [1,2-a]- benzimidazole,
et leurs sels d'addition avec un acide.

Parmi les dérivés imidazolo-azoliques utilisables a titre de coupleurs hétérocycliques dans la composition tinctoriale conforme à l'invention, on peut plus particulièrement citer les composés décrits dans les demandes de brevets et brevets suivants : US 5,441,863 ; JP 62-279 337 ; JP 06-236 011 et JP 07-092 632, dont les enseignements font partie intégrante de la présente demande.

A titre de dérivés imidazolo-azoliques, on peut tout particulièrement eiter :
- le 7,8-dicyano-imidazolo- [3,2-a]- imidazole,
- le 7,8-dicyano-4-méthyl-imidazolo- [3,2-a]- imidazole,
et leurs sels d'addition avec un acide.

Parmi les dérivés pyrazolo-pyrimidiniques utilisables à titre de coupleurs hétérocycliques dans la composition tinctoriale conforme à l'invention, on peut plus particulièrement citer les composés décrits dans la demande de brevet EP-A-0 304 001 dont l'enseignement fait partie intégrante de la présente demande.

A titre de dérivés pyrazolo-pyrimidiniques, on peut tout particulièrement citer :
- le pyrazolo [1,5-a] pyrimidin-7-one,
- le 2,5-diméthyl pyrazolo [1,5-a] pyrimidin-7-one,
- le 2-méthyl-6-éthoxycarbonyl pyrazolo [1,5-a] pyrimidin-7-one,
- le 2-méthyl-5-méthoxyméthyl pyrazolo [1,5-a] pyrimidin-7-one,
- le 2-ter-butyl-5-trifluorométhyl pyrazolo [1,5-a] pyrimidin-7-one,
- 2,7-diméthyl pyrazolo [1,5-a] pyrimidin-5-one,
et leurs sels d'addition avec un acide.

Parmi les dérivés de pyrazolin-3,5-diones utilisables à titre de coupleurs hétérocycliques dans la composition tinctoriale conforme à l'invention, on peut plus particulièrement citer les composés décrits dans les demandes de brevets et brevets suivants : JP 07-036159, JP 07-084348 et US 4 128 425, et dans les publications suivantes :
- L. WYZGOWSKA, Acta. Pol. Pharm. 1982, 39 (1-3), 83.
- E. HANNIG, Pharmazie, 1980, 35 (4), 231
- M. H. ELNAGDI, Bull. Chem. Soc. Jap., 46 (6), 1830, 1973
- G. CARDILLO, Gazz, Chim. Ital. 1966, 96, (8-9), 973 ;
dont les enseignements font partie intégrante de la présente demande.

A titre de dérivés de pyrazolin-3,5-diones, on peut tout particulièrement citer :
- la 1,2-diphényl pyrazolin-3,5-dione,
- la 1,2-diéthyl pyrazolin-3,5-dione,
et leurs sels d'addition avec un acide.

Parmi les dérivés pyrrolo-[3,2-d]-oxazoliques utilisables à titre de coupleurs hétérocycliques dans la composition tinctoriale conforme à l'invention, on peut plus particulièrement citer les composés décrits dans la demande de brevet JP 07 325 375 dont l'enseignement fait partie intégrante de la présente demande.

Parmi les dérivés pyrzolo-[3,4-d]-thiazoliques utilisables à titre de coupleurs hétérocycliques dans la composition tinctoriale conforme à l'invention, on peut plus particulièrement citer les composés décrits dans la demande de brevet JP 07 244 361 et dans J. Heterocycl. Chem. 16, 13, (1979).

Parmi les dérivés S-oxyde-thiazolo-azoliques et S,S-dioxyde-thiazolo-azoliques utilisables à titre de coupleurs hétérocycliques dans la composition tinctoriale conforme à l'invention, on peut plus particulièrement citer les composés décrits dans les documents suivants :
- JP 07 098489 ;
- Khim. Geterotsilk. Soedin, 1967, p. 93 ;
- J. Prakt. Chem., 318, 1976, p. 12 ;
- Indian J. Heterocycl. Chem. 1995, 5 (2), p. 135 ;
- Acta. Pol. Pharm. 1995, 52 (5), 415 ;
- Heterocycl. Commun. 1995, 1 (4), 297 ;
- Arch. Pharm. (Weinheim, Ger.), 1994, 327 (12), 825.

Parmi les naphtols et naphtols acylés utilisables à titre de coupleurs dans la composition tinctoriale conforme à l'invention, on utilisera de préférence les composés de formule (X) suivante, et leurs sels d'addition avec un acide : 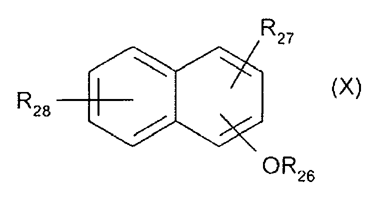 dans laquelle:
- R₂₆ représente un atome d'hydrogène ou un groupement -CO-R dans lequel R représente un radical alkyle en C₁-C₄ ;
- R₂₇ représente un atome d'hydrogène, un radical hydroxyle, alkyle en C₁-C₄, ou un groupement -SO₃H ;
- R₂₈ représente un atome d'hydrogène, ou un radical hydroxyle ; étant entendu qu'au moins un des radicaux R₂₆ à R₂₈ est différent d'un atome d'hydrogène.

Parmi les naphtols et naphtols acylés de formule (X), utilisables à titre de coupleurs dans la composition tinctoriale conforme à l'invention, on peut notamment citer le 1,7-dihydroxy naphtalène, le 2,7-dihydroxy naphtalène, le 2,5-dihydroxy naphtalène, le 2,3-dihydroxy naphtalène, le 1-acétoxy 2-méthyl naphtalène, le 1-hydroxy 2-méthyl naphtalène, l'acide 1-hydroxy 4-naphtalène sulfonique, et leurs sels d'addition avec un acide.

Lorsqu'ils sont présents, le ou les coupleurs représentent de préférence de 0,0001 à 10 % en poids environ du poids total de la composition tinctoriale et encore plus préférentiellement de 0,005 à 5 % en poids environ de ce poids.

La composition tinctoriale conforme à l'invention peut encore contenir, de plues au moins une base d'oxydation additionnelle, qui peut être choisie parmi les bases d'oxydation classiquement utilisées en teinture d'oxydation et parmi lesquelles on peut notamment eiter, les bis-phénylalkylènediamines, les para-aminophénols, les orthoaminophénols et les bases hétérocycliques, et leurs sels d'addition avec un acide.

Selon un mode de réalisation particulier, la base additionnelle est choisie parmi les bases d'oxydation hétérocycliques, les bases doubles, les paraaminophénols substitués, les orthoaminophénols, les dérivés de paraphénylènediamine de formule (II) suivante, et leurs sels d'addition avec un acide : 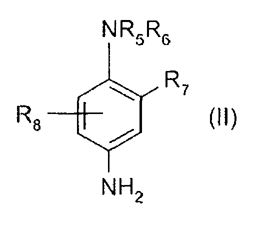 dans laquelle :
- R₅ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un ou plusieurs groupements azotés, phényle ou 4'-aminophényle ;
- R₆ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄) ou alkyle en C₁-C₄ substitué par un ou plusieurs groupements azotés ;
- R₇ représente un atome d'hydrogène, un atome d'halogène tel qu'un atome de chlore, de brome, d'iode ou de fluor, un radical alkyle en C₁-C₄, monohydroxyalkyle en C₁-C₄, hydroxyalcoxy en C₁-C₄, acétylaminoalcoxy en C₁-C₄, mésylaminoalcoxy en C₁-C₄ ou carbamoylaminoalcoxy en C₁-C₄,
- R₈ représente un atome d'hydrogène, d'halogène ou un radical alkyle en C₁-C₄.

Parmi les paraphénylènediamines de formule (II) utilisables à titre de base d'oxydation additionnelle dans la composition tinctoriale conforme à l'invention, on peut plus particulièrement citer la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl) amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

Parmi les paraphénylènediamines de formule (II) ci-dessus, on préfère tout particulièrement la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-β-acétylaminoéthyloxy paraphénylénediamine, et leurs sels d'addition avec un acide.

Encore plus préférentiellement, on préfère parmi les paraphénylènediamines de formule (11) ci-dessus, la 2-β-hydroxyéthyl paraphénylènediamine, la N,N-bis-(β-hydroxyéthyl) paraphénylènediamine, et leurs sels d'addition avec un acide.

Selon l'invention, on entend par bases doubles, les composés comportant au moins deux noyaux aromatiques sur lesquels sont portés des groupements amino et/ou hydroxyle.

Parmi les bases doubles utilisables à titre de base d'oxydation additionnelle dans la composition tinctoriale conforme à l'invention, on peut notamment citer les bases doubles de formule (III) suivante, et leurs sels d'addition avec un acide : 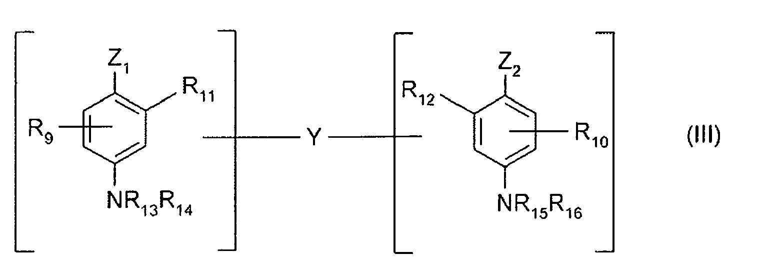 dans laquelle:
- Z₁ et Z₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₉ et R₁₀ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₁₁, R₁₂, R₁₃, R₁₄, R₁₅ et R₁₆, identiques ou différents, représentent un atome d'hydrogène, un bras de liaison Y ou une radical alkyle en C₁-C₄ ;
étant entendu que les bases doubles de formule (III) ne comportent qu'un seul bras de liaison Y par molécule.

Parmi les groupements azotés présents dans la base double de formule (III) ci-dessus, on peut citer notamment les radicaux amino, monoalkyl(C₁-C₄)amino, dialkyl(C₁-C₄)ainino, trialkyl(C₁-C₄)amino, monohydroxyalkyl(C₁-C₄)amino, imidazolinium et ammonium.

Parmi les bases doubles de formule (III) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminpophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi ces bases doubles de formule (III), le N,N'-bis-(β-hydroxyéthyl) N,N'-bis-(4'-aminophényl) 1.3-diamino propanol, le 1,8-bis-(2,5-diaminophénoxy)-3,5-dioxaoctane ou l'un de leurs sels d'addition avec un acide sont particulièrement préfèrés.

Parmi les para-aminophénols substitués utilisables à titre de seconde base d'oxydation dans la composition tinctoriale conforme à l'invention, on peut notamment citer les para-aminophénols substitués de formule (IV) suivante, et leurs sels d'addition avec un acide : 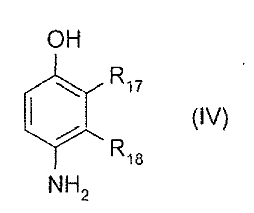 dans laquelle :
- R₁₇ représente un atome d'hydrogène ou d'halogène, un radical alkyle en C₁-C₄, monohydroxyalkyle en C₁-C₄, alcoxy(C₁-C₄)alkyle(C₁-C₄), aminoalkyle en C₁-C₄ ou hydroxyalkyl(C₁-C₄)ammoalkyle en C₁-C₄,
- R₁₈ représente un atome d'hydrogène ou d'halogène, un radical, alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
étant entend qu'au moins un des radicaux R₁₇ ou R₁₈ est différent d'un atome d'hydrogène.

Parmi les para-aminophénols de formule (IV) ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino, 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les orthoaminophénols utilisables à titre de bases d'oxydation additionnelle dans les compositions tinctoriales conformes à l'invention, on peut plus particulièrement citer le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques utilisables à titre de bases d'oxydation additionnelle dans les compositions tinctoriales conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets DE 2 359 399 ; JP 88-169 571 ; JP 05 163 124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 6-hydroxy 2,4,5-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol; le 3-amino pyrazolo-[1,5-a]-pyrimidin-5-ol; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydrox-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine, leurs sels d'addition avec un acide, et leurs formes tautomères, lorsqu'il existe un équilibre tautomérique.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4.5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 1-(β-hydroxyéthyl) pyrazole, le 4,5-diamino 3-méthyl 1-phényl pyrazole, le 4.5-diamino 1-méthyl 3-phényl pyrazole, le 4-amino 1,3-diméthyl 5-hydrazino pyrazole, le 1-benzyl 4,5-diamino 3-méthyl pyrazole, le 4.5-diamino 3-tert-butyl 1-méthyl pyrazole, le 4.5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4.5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Lorsqu'elles sont utilisées, la ou les bases d'oxydation additionnelles représentent de préférence de 0,0005 à 12 % en poids environ du poids total de la composition tinctoriale, et encore plus préférentiellement de 0,005 à 6 % en poids environ de ce poids.

D'une manière générale, les sels d'addition avec un acide utilisables dans le cadre des compositions tinctoriales de l'invention (composés de formule (1), coupleurs et bases d'oxydation additionnelles) sont notamment choisis parmi les chlorhydrates, les bromhydrates, les sulfates, les citrates, les succinates, les tartrates, les lactates, les phosphates et les acétates.

La composition tinctoriale conforme à l'invention peut en outre renfermer un ou plusieurs colorants directs. Ce colorant direct peut être un colorant direct synthétique choisi parmi les azoïques, les quinoniques, les triarylméthaniques, les indoaminiques ou les aziniques et/ou un colorant naturel. Les colorants directs synthétiques utilisables Selon l'invention peuvent être non ioniques, anioniques ou cationiques.

Parmi les colorants directs synthétiques azoïques utilisables selon l'invention, on peut citer les colorants azoïques cationiques décrits dans les demandes de brevets WO 95/15144, WO-95/01772 et EP-714954 dont le contenu fait partie intégrante de l'invention.

On peut également citer parmi les colorants directs azoïques les colorants suivants, décrits dans le COLOUR INDEX INTERNATIONAL 3^{e} édition : Disperse Red 17, Acid Yellow 9, Acid Black 1, Basic Red 22, Basic Red 76, Basic Yellow 57, Basic Brown 16, Acid Yellow 36, Acid Orange 7, Acid Red 33, Acid Red 35, Basic Brown 17, Acid Yellow 23, Acid Orange 24, Disperse Black 9. On peut également citer parmi eux, le 1-(4'-aminodiphénylazo)-2-méthyl-4-bis-(β-hydroxyéthyl)-aminobenzéne et l'acide 4-hydroxy-3-(2-méthoxyphénylazo)-1-naphtalène sulfonique.

Parmi les colorants directs synthétiques quinoniques, on peut citer les suivants : Dispense Red 15, Souvent Violet 13, Acid Violet 43, Disperse Violes 1, Disperse Violet 4, Disperse Blue 1, Disperse Violet 8, Disperse Blue 3, Disperse Red 11, Acid Blue 62, Disperse Blue 7, Basic Blue 22, Disperse Violet 15, Basic Blue 99, ainsi que les composés :
- 1-N-méthylmorpholiniumpropylamino-4-hydroxyanthraquinone,
- 1-aminopropylamino-4-méthylaminoanthraquinone,
- 1-aminopropylaminoanthraquinone,
- 5-β-hydroxyéthyl-1,4-diaminoanthraquinone,
- 2-aminoéthylaminoanthraquinone,
- 1,4-bis-(β,γ-dihydroxypropylamino)-anthraquionone.

Parmi les colorants directs synthétiques aziniques, on peut citer les suivants : Basic Blue 17, Basic Red 2.

Parmi les colorants directs synthétiques triarylméthaniques utilisables selon l'invention, on peut citer les suivants : Basic Green 1, Acid blue 9, Basic Violet 3, Basic Violet 14, Basic Blue 7, Acid Violet 49, Basic Blue 26, Acid Blue 7.

Parmi les colorants directes synthétiques indoaminiques utilisables selon l'invention, on peut citer les suivants :
- 2-β-hydroxyéthylamino-5-[bis-(β-4'-hydroxyéthyl)-amino]-anilino-1,4-benzoquinone
- 2-β-hydroxyéthylamino-5-(2'-méthoxy-4'-amino)-anilino-1,4-benzoquinone
- 3-N(2'-chloro-4'-hydroxy)-phényl-acétylamino-6-méthoxy-1,4-benzoquinone imine
- 3-N(3'-chloro-4,-méthylamino)-phényl-uréido-6-méthyl-1,4-benzoquinone imine
- 3-[4'-N(éthyl,carbamylméthyl)-amino]phényl-uréido-6-éthyl-1,4-benzoquionone imine.

Par colorant naturel, on entend au sens de l'invention, les composés qui existent dans la nature qu'ils aient été obtenus par extraction ou reproduits via la chimie. Parmi les colorants directs naturels utilisables Selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoction contenant ces colorants naturels et notamment les cataplasmes ou extraits à base de henné.

Le ou les colorants directs synthétiques selon l'invention et/ou le zou les colorants naturels représentent de 0,001 à 20% en poids environ du poids total de la composition et encore plus préférentiellement de 0,005 à 10% en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotéres, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrant, des parfums, des tampons, des agents dispersants, des agents de conditionnement tels que par exemple des silicones volati les ou non volatiles, modifiées ou non modifiées, des agents filmogènes, des céramides, des agents conservateurs, des agents opacifiants.

Selon un mode de réalisation particulier, la composition tinctoriale de l'invention comprend au moins un polymère choisi parmi :
- (i)les polymères amphotères,
- (ii)les polymères cationiques contenant des motifs récurrents de structures (II) ou (III) suivantes, et,
- (iii)les polymères différents des précédents qui sont amphiphiles et comportent au moins une chaîne grasse,
- Structure (II) : 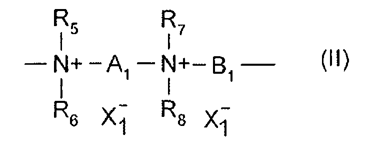 dans laquelle :
   R₅, R₆, R₇ et R₈, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₅, R₆, R₇ et R₈, ensemble ou séparément, constituent avec les atomes d'azote auxquels ils sont rattachés des hétérocycles contenant éventuellement un second hétéroatome autre que l'azote ou bien R₅, R₆, R₇ et R₈ représentent, un radical alkyle en C₁-C₆ linéaire ou ramifié substitué par un groupement nitrile, ester, acyle, amide ou -CO-O-R₁₃-D ou -CO-NH-R₁₃-D où R₁₃ est un alkylène et D un groupement ammonium quaternaire ;
   A₁ et B₁ représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatique, ou un ou plusieurs atomes d'oxygène, de soufre ou des groupements sulfoxyde, sulfone, disulfure, amino, alkylamino, hydroxyle, ammonium quaternaire, uréido, amide ou ester.
   A₁, R₅ et R₇ peuvent former avec les deux atomes d'azote auxquels ils sont rattachés un cycle pipérazinique ; en outre si A₁ désigne un radical alkylène ou hydroxyalkylène linéaire ou ramifié, saturé ou insaturé, B1 peut également désigner un groupement - (CH₂)ₙ-CO-T-OC-(CH₂)n- dans lequel n est compris entre 1 et 100 et de préférence entre 1 et 50, et T désigne :
      a) un reste de glycol de formule : -O-Z-O-, où Z désigne un radical hydrocarboné linéaire ou ramifié ou un groupement répondant à l'une des formules suivantes :

         -(CH₂-CH₂-O)x-CH₂-CH₂-

         -[CH₂-CH(CH₃)-O]y-CH₂-CH(CH₃)-

         où x et y désignent un nombre entier de 1 à 4, représentant un degré de polymérisation défini et unique, ou un nombre quelconque de 1 à 4 représentant une degré de polymérisation moyen ;
      b) une reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent

         -CH₂-CH₂-S-S-CH₂-CH₂- ;
      d) un groupement uréyléne de formule : -NH-CO-NH- ;
   X₁ est un anion dérivé d'un acide minéral ou organique et de préférence le chlore ou le brome;
- Structure (III) 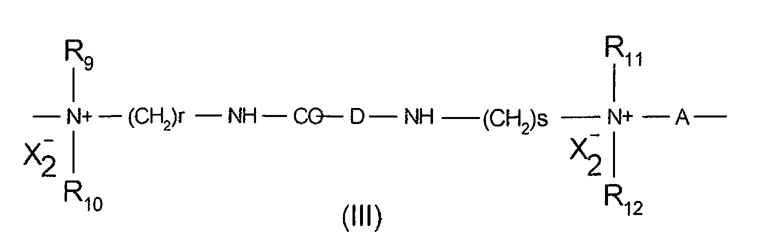 dans laquelle :
   R₉, R₁₀, R₁₁ et R_{12,} identiques ou différents, représentent un atome d'hydrogène ou un radical méthyle, éthyle, propyle, β-hydroxyétliyle, β-hydroxypropyle ou -CH₂CH₂ (OCH₂CH₂)pOH, où p est égal à 0 ou à un nombre entier compris entre 1 et 6,
   sous réserve que R₉, R₁₀, R₁₁ et R₁₂ ne représentent pas simultanément un atome d'hydrogène,
   r et s, identiques ou différents, sont des nombres entiers compris entre 1 et 6,
   D est nul ou désigne un groupement -(CH₂)_{q}-CO- dans lequel q est nul ou égal à un nombre entier compris entre 1 et 34,
   A désigne le radical d'un dihalogénure ou représente de préférence -CH₂-CH₂-O-CH₂-CH₂-,
   X₂ désigne un anion dérivé d'un acide minéral ou organique, de préférence un atome d'halogène.

Les polymères cationiques à motifs récurrents de structure (II) ont de préférence une masse moléculaire moyenne en nombre généralement comprise entre 1000 et 100000. Des polymères de ce type sont notamment décrits dans tes brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.

Selon l'invention, parmi ces polymères cationiques à motifs de structure (II), on utilise plus particulièrement ceux constitués de motifs récurrents répondant à la structure (IV) suivante: 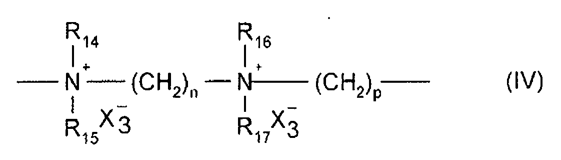 dans laquelle R₁₄, R₁₅, R₁₆ et R₁₇, identiques ou différents, désignent un radical alkyle ou hydroxyalkyle ayant de 1 à 4 atomes de carbone environ, n et p sont des nombres entiers variant de 2 à 20 environ et, X₃⁻ est un anion dérivé d'un acide minéral ou organique.

Plus particulièrement, on préfère encore utiliser le polymère à motifs de structure (IV) dans laquelle R₁₄, R₁₅, R₁₆, R₁₇ désignent le radical méthyle, n et p sont respectivement égaux à 6 et 3 et X₃⁻ est égal à Cl⁻ ; ce polymère a pour nom INCI : HEXADIMETHRINE CHLORIDE.

Les polymères cationiques à motifs récurrents de structure (III) sont notamment décrits dans la demande de brevet EP-A-122 324 et peuvent être préparés selon les procédés décrits dans les brevets U.S.A. N° 4 157 388, 4 390 689, 4 702 906, 4 719 282.

Parmi ces polymères, on préfère selon l'invention utiliser plus particulièrement ceux constitués de motifs récurrents répondant à la structure (V) suivante:

-[-N⁺(CH₃)₂-(CH₂)ᵣ-NH-CO-D-NH-(CH₂)ᵣ-N⁺(CH₃)₂-(CH₂)₂

-O-(CH₂)₂-]- 2 X⁻ (V)

dans laquelle r désigne un nombre entier variant de 1 à 6 environ, D peut être nul ou peut représenter un groupement -(CH₂)_{q}-CO- dans lequel q désigne un nombre égal à 4 ou à 7, et X⁻ est un anion dérivé d'un acide minéral ou organique, et de préférence de masse moléculaire mesurée par RMN du Carbone 13 inférieure à 100000.

Parmi les polymères cationiques de structure (V), on préfère plus particulièrement encore ceux pour lesquels :
a) D représente un groupement -(CH₂)₄-CO-, X désigne un atome de chlore, la masse moléculaire mesurée par RMN du Carbone 13 (RMN¹³C) étant d'environ 5600 ; un polymère de ce type est proposé par la société MIRANOL sous le nom de MIRAPOL-ADI.
b) D représente un groupement -(CH₂)₇-CO-, X désigne un atome de chlore, la masse moléculaire mesurée par RMN du Carbone 13 ( RMN¹³C ) étant d'environ 8100 ; un polymère de ce type est proposé par la société MIRANOL sous le nom de MIRAPOL-AZI,
c) D désigne la valeur zéro, X désigne un atome de chlore, la masse moléculaire mesurée par RMN du Carbone 13, ( RMN¹³C ) étant d'environ 25500 ; un polymère de ce type est vendu par la société MIRANOL sous le nom MIRAPOL-A15,
d) un " Block Copolymer " formé de motifs correspondant aux polymères décrits aux alinéas a) et c), proposé par la société MIRANOL sous les noms MIRAPOL-9, (masse moléculaire RMN¹³C, environ 7800) MIRAPOL-175, (masse moléculaire RMN¹³C, environ 8000) MIRAPOL-95, (masse moléculaire RMN¹³C, environ 12500). Plus particulièrement encore, on préfère selon l'invention le polymère de formule (V) dans laquelle D est nul, X désigne un atome de chlore, la masse moléculaire mesurée par RMN du Carbone 13, ( RMN¹³C ) étant d'environ 25500.

Selon l'invention, le ou les polymères cationiques peuvent représenter de 0,01 à 10% en poids environ du poids total de la composition, de préférence de 0,05 à 5% et plus préférentiellement encore de 0,1 à 3%.

Les polymères amphotères utilisables conformément à la présente invention peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes;
K et M peuvent également désigner une chaîne polymère cationique comportant des groupements amine primaire, secondaire, tertiaire ou quaternaire, dans laquelle au moins l'un des groupements aminé porte une groupement carboxylique ou sulfonique relié par l'intermédiaire d'un radical hydrocarboné, ou bien K et M font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères amphotères plus particulièrement préférés sont choisis parmi les polymères suivants :
(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537. On peut également citer le copolymère acrylate de sodium / chlorure d'acrylamidopropyl trimethyl ammonium vendu sous la dénomination POLYQUART KE 3033 par la Société HENKEL.
   Le composé vinylique substitué contenant au moins un atome basique peut être également un sel de dialkyldiallylammonium tel que le chlorure de diméthyldiallylammonium. Les copolymères d'acide acrylique et de ce dernier monomère sont proposés sous les appellations MERQUAT 280, MERQUAT 295 et MERQUAT PLUS 3330 par la société CALGON.
(2) Les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants aminé primaire, secondaire, tertiaire et quaternaire des acides acrylique et méthacrylique et le produit de quaternisation du méthacrylate de diméthytaminoéthyle avec le sulfate de diméthyle ou diéthyle.
   Les acrylamides ou méthacrylamides N-substitués plus paticulièrement préférés selon l'invention sont les groupements dont les radicaux alkyle contiennent de 2 à 12 atomes de carbone et plus particulièrement le N-éthylacrylamide, le N-tertiobutyl-acrylamide, le N-tertiooctyl-acrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, protonique, itaconique, maléique, fumarique ainsi que les monoesters d'alkyle ayant 1 à 4 atomes de carbone des acides ou des anhydrides maléique ou fumarique.
   Les comonomères basiques préférés sont des méthacrylates d'aminoéthyle, de butyl aminoéthyle, de N,N'-diméthylaminoéthyle, de N-tertio-butylaminoéthyle.
   On utilise particulièrement les copolymères dont la dénomination CTFA (4ème Ed., 1991) est Octylacrylamide/acrylates/butylaminoethylmethacrylate copolymer tels que les produits vendus sous la dénomination AMPHOMER ou LOVOCRYL 47 par la société NATIONAL STARCH.
(3) Les polyaminoamides réticulés et alcoylés partiellement ou totalement dérivant de polyaminoamides de formule générale :

   ⁅CO-R₁₈-CO-Z⁆ (VI)

   dans laquelle R₁₈ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical 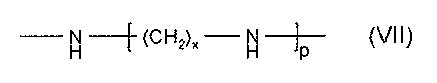 où x=2 et p=2 ou 3, ou bien x=3 et p=2
      ce radical dérivant de la diéthylène triamine, de la triéthylène tétraamine ou de la dipropylène triamine;
   b) dans les proportions de 0 à 40 moles % le radical (VII) ci-dessus, dans lequel x=2 et p=1 et qui dérive de l'éthylènediamine, ou le radical dérivant de la pipérazine : 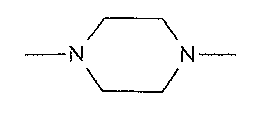
   c) dans les proportions de 0 à 20 moles % le radical -NH-(CH₂)₆-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.

   Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.
   Les alcanes sultones utilisées dans l'alcoylation sont de préférence la propane ou la butane sultone, les sels des agents d'alcoylation sont de préférence les sels de sodium ou de potassium.
(4) Les polymères comportant des motifs zwittérioniques de formule : 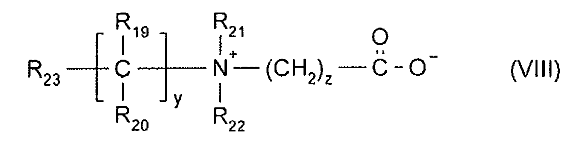 dans laquelle R₂₃ désigne un groupement insaturé polymérisable tel qu'un groupement acrylate, méthacrylate, acrylamide ou méthacrylamide, y et z représentent un nombre entier de 1 à 3, R₁₉ et R₂₀ représentent un atome d'hydrogène, méthyle, éthyle ou propyle, R₂₁ et R₂₂ représentent un atome d'hydrogène ou un radical alkyle de telle façon que la somme des atomes de carbone dans R₂₁ et R₂₂ ne dépasse pas 10.
   Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
   A titre d'exemple, on peut citer le copolymère de méthacrylate de butyle / méthacrylate de diméthylcarboxyméthylammonio-éthyle tel que le produit vendu sous la dénomination DIAFORMER Z301 par la société SANDOZ.
(5) les polymères dérivés du chitosane comportant des motifs monomères répondant aux formules (IX), (X), (XI) suivantes : 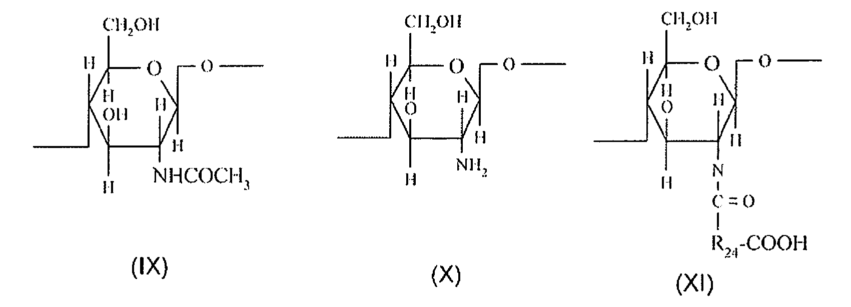 le motif (IX) étant présent dans des proportions comprises entre 0 et 30%, le motif (X) dans des proportions comprises entre 5 et 50% et le motif (XI) dans des proportions comprises entre 30 et 90%, étant entendu que dans ce motif(XI), R₂₄ représente un radical de formule : 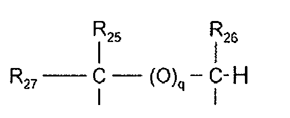 dans laquelle
   si q=0, R₂₅, R₂₆ et R₂₇, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux R₂₅, R₂₆ et R₂₇ étant dans ce cas un atome d'hydrogène ;
   ou si q=1, R₂₅, R₂₆ et R₂₇ représentent chacun un atome d'hydrogène, ainsi que les sels formés par ces composés avec des bases ou des acides.
(6) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.
**(7)** Les polymères répondant à la formule générale (XII) tels que ceux décrits par exemple dans le brevet français 1 400 366 : 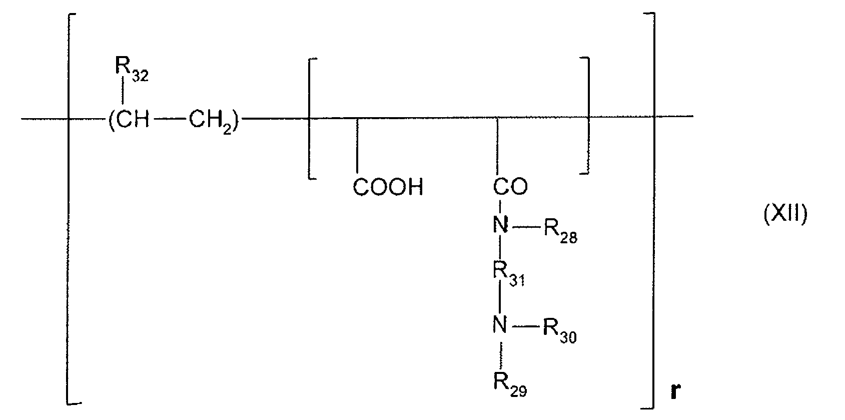 dans laquelle R₃₂ représente un atome d'hydrogène, un radical CH₃O, CH₃CH₂O, phényle, R₂₈ désigne l'hydrogène ou un radical alkyle inférieur tel que méthyle, éthyle, R₂₉ désigne l'hydrogène ou un radical alkyle inférieur tel que méthyle, éthyle, R₃₀ désigne un radical alkyle inférieur tel que méthyle, éthyle ou un radical répondant à la formule: -R₃₁-N(R₂₉)₂, R₃₁ représentant un groupement -CH₂-CH₂-, -CH₂-CH₂-CH₂-,-CH₂-CH(CH₃)- , R₂₉ ayant les significations mentionnées ci-dessus,
   ainsi que les homologues supérieurs de ces radicaux et contenant jusqu'à 6 atomes de carbone,
   r est tel que le poids moléculaire est compris entre 500 et 6000000 et de préférence entre 1000 et 1000000.
**(8)** Des polymères amphotères du type -D-X-D-X- choisis parmi:
   a) les polymères obtenus par action de l'acide chloracétique ou le chloracétate de sodium sur les composés comportant au moins un motif de formule:

      -D-X-D-X-D- **(XIII)**

      ou D désigne un radical 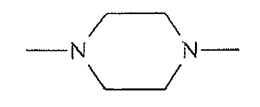 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;
   b) les polymères de formule :

      -D-X-D-X- **(XIV)**

      où D désigne un radical 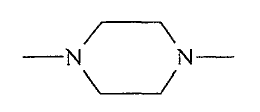 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou prieurs fonctions hydroxyle et bétaïnisées par réaction avec l'acide chloracétique ou du chloracécate de soude.
**(9)** Les copolymères alkyl(C₁-C₅)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonoméres vinyliques tels que le vinylcaprolactame.

Les polymères amphotères particulièrement préférés selon l'invention sont ceux de la famille (1).

Selon l'invention, le ou les polymères amphotères peuvent représenter de 0,01 % à 10 % en poids, de préférence de 0,05 % à 5 % en poids, et encore plus préférentiellement de 0,1 % à 3 % en poids, du poids total de la composition.

Les polymères différents des précédents qui sont amphiphiles et comportent au moins une chaîne grasse utiles dans la présente intention sont également appelés polymères associatifs; ils peuvent être de type non ionique, anionique, ou cationique.

Parmi les polymères amphiphiles comportant au moins une chaîne grasse et de type anionique, on peut citer :
- **(I)** ceux comportant au moins un motif hydrophile, et au moins un motif éther d'allyle à chaîne grasse, plus particulièrement ceux dont le motif hydrophile est constitué par un monomère anionique insaturé éthylénique, plus particulièrement encore par un acide carboxylique vinylique et tout particulièrement par un acide acrylique ou un acide méthacrylique ou les mélanges de ceux ci, et dont le motif éther d'allyle à chaîne grasse correspond au monomère de formule (XV) suivante :

   CH₂ = C R' CH₂ O Bₙ R (XV)

   dans laquelle R' désigne H ou CH₃, B désigne le radical éthylèneoxy, n est nul ou désigne un entier allant de 1 à 100, R désigne un radical hydrocarboné choisi parmi les radicaux alkyl, arylalkyle, aryle, alkylaryle, cycloalkyle, comprenant de 8 à 30 atomes de carbone, de préférence 10 à 24, et plus particulièrement encore de 12 à 18 atomes de carbone. Un motif de formule (XV) plus particulièrement préféré est un motif dans lequel R' désigne H, n est égal à 10, et R désigne un radical stéaryl (C₁₈).
   Des polymères amphiphiles anioniques de ce type sont décrits et préparés, selon un procédé de polymérisation en émulsion, dans le brevet EP-0 216 479.
   Parmi ces polymères anioniques à chaîne grasse, on préfère particulièrement selon l'invention, les polymères formés à partir de 20 à 60% en poids d'acide acrylique et/ou d'acide méthacrylique, de 5 à 60% en poids de (méth)acrytates d'alkyles inférieurs, de 2 à 50% en poids d'éther d'allyl à chaîne grasse de formule (XV), et de 0 à 1% en poids d'un agent réticulant qui est un monomère insaturé polyéthylénique copolymérisable bien connu, comme le phtalate de diallyle, le (méth)acrytate d'allyl, le divinylbenzène, le diméthacrylate de (poly)éthylèneglycol, et le méthylène-bis-acrylamide.
   Parmi ces derniers, on préfère tout particulièrement les terpolymères réticulés d'acide méthacrylique, d'acrylate d'éthyle, de polyéthylèneglycol (10 OE) éther d'alcool stéarylique (Steareth 10), notamment ceux vendus par la société ALLIED COLLOIDS sous les dénominations SALCARE SC 80 et SALCARE SC90 qui sont des émulsions aqueuses à 30% d'un terpolymère réticulé d'acide méthacrylique, d'acrylate d'éthyle et de steareth-10-allyl éther (40/50/10).
- **(II)** ceux comportant au moins un motif hydrophile de type acide carboxylique insaturé oléfinique, et au moins un motif hydrophobe de type ester d'alkyl (C₁₀-C₃₀) d'acide carboxylique insaturé.
   De préférence, ces polymères sont choisis parmi ceux dont le motif hygrophile de type acide carboxylique insaturé oléfinique correspond au monomère de formule (XVI) suivante : 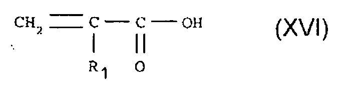 dans laquelle, R₁ désigne H ou CH₃ ou C₂H₅, c'est-à-dire des motifs acide acrylique, acide méthacrylique ou acide éthacrylique, et dont le motif hydrophobe de type ester d'alkyl (C₁₀-C₃₀) d'acide carboxylique insaturé correspond au monomère de formule (XVII) suivante : 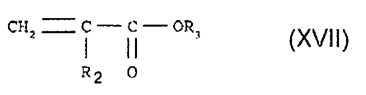 dans laquelle, R₂ désigne H ou CH₃ ou C₂H₅ (c'est-à-dire des motifs acrylates, méthacrylates ou éthacrylates) et de préférence H (motifs acrylates) ou CH₃ (motifs méthacrylates), R₃ désignant un radical alkyle en C₁₀-C₃₀, et de préférence en C₁₂-C₂₂.
   Des esters d'alkyles (C₁₀-C₃₀) d'acides carboxyliques insaturés conformes à l'invention comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.
   Des polymères anioniques de ce type sont par exemple décrits et préparés, selon les brevets US-3 915 921 et 4 509 949.
   Parmi ce type de polymères anioniques à chaîne grasse, on utilisera plus particulièrement des polymères formés à partir d'un mélange de monomères comprenant:
   (i) essentiellement de l'acide acrylique,
   (ii) un ester de formule (XVI) décrite ci-dessus et dans laquelle R₂ désigne H ou CH₃, R₃ désignant un radical alkyle ayant de 12 à 22 atomes de carbone,
   (iii) et un agent réticulant, qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.

   Parmi ce type de polymères anioniques à chaîne grasse, on utilisera plus particulièrement ceux constitués de 95 à 60% en poids d'acide acrylique (motif hydrophile), 4 à 40% en poids d'acrylate d'alkyles en C₁₀-C₃₀ (motif hydrophobe), et 0 à 6% en poids de monomère polymérisable réticulant, ou bien ceux constitués de 98 à 96% en poids d'acide acrylique (motif hydrophile), 1 à 4% en poids d'acrylate d'alkyles en C₁₀-C₃₀ (motif hydrophobe), et 0,1 à 0,6% en poids de monomère polymérisable réticulant tel que ceux décrits précédemment.
   Parmi lesdits polymères ci-dessus, on préfère tout particulièrement selon la présente invention, les produits vendus par la société GOODRICH sous les dénominations commerciales PEMULEN TR1, PEMULEN TR2, CARBOPOL 1382, et encore plus préférentiellement le PEMULEN TR1, et le produit vendu par la société S.E.P.P.I.C. sous la dénomination COATEX SX .
- **(III)** les terpolymères d'anhydride maléique/α-oléfine en C₃₀-C₃₈ maléate d'alkyle tel que le produit (copolymère anhydride maléique/α-oléfine en C₃₀-C₃₈/maléate d'isopropyle) vendu sous le nom PERFORMA V 1608 par la société NEWPHASE TECHNOLOGIES.
- **(IV)** les terpolymères acryliques comprenant :
   (a) environ 20% à 70% en poids d'un acide carboxylique à insaturation α,β-monoéthylénique,
   (b) environ 20 à 80% en poids d'un monomère à insaturation α,β-monocthyiénique non-tensio-actif différent de (a),
   (c) environ 0,5 à 60% en poids d'un mono-uréthane non-ionique qui est le produit de réaction d'un tensio-actif monohydrique avec un monoisocyanate à insaturation monoéthylénique,
   tels que ceux décrits dans la demande de brevet EP-A-0173109 et plus particulièrement celui décrit dans l'exemple 3, à savoir, un terpolymère acide méthacrylique /acrylate de méthyle/diméthyl métaisopropényl benzyl isocyanate d'alcool béhényle éthoxylé (40OE) en dispersion aqueuse à 25%.
- **(V)** les copolymères comportant parmi leurs monomères un acide carboxylique à insaturation α,β-monoéthylénique et un ester d'acide carboxylique à insaturation α,β-monoéthylénique et d'un alcool gras (C8-C30)oxyalkyléné.

Préférentiellement ces composés comprennent également comme monomère un ester d'acide carboxylique à insaturation α,β-monoéthylénique et d'alcool en C1-C4.

A titre d'exemple de ce type de composé on peut citer l'ACULYN 22 vendu par la société ROHM et HAAS, qui est un terpolymère acide méthacrylique/acrylate d'éthyle/méthacrylate de stéaryle oxyalkyléné.

Les polymères amphiphiles à chaîne grasse et de type non ionique, utilisés selon l'invention, sont choisis de préférence parmi :
- **(1)** les celluloses modifiées par des groupements comportant au moins une chaîne grasse ;
   on peut citer à titre d'exemple :
   - les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en C₈-C₂₂, comme le produit NATROSOL PLUS GRADE 330 CS (alkyles en C₁₆) vendu par la société AQUALON, ou le produit BERMOCOLL EHM 100 vendu par la société BEROL NOBEL,
   - celles modifiées par des groupes polyalkylène glycol éther d'alkyl phénol, tel que le produit AMERCELL POLYMHR HM-1500 (polyéthylène glycol (15) éther de nonyl phénol) vendu par la société AMERCHOL.
- **(2)** les hydroxypropylguars modifiés par des groupements comportant au moins une chaîne grasse tel que le produit ESAFLOR HM 22 (chaîne alkyle en C₂₂) vendu par la société LAMBERTI, les produits RE210-18 (chaîne alkyle en C₁₄) et RE205-J (chaîne alkyle en C₂₀) vendus par la société RHONE POULENC.
- **(3)** les copolymères de vinyl pyrrolidone et de monomères hydrophobes à chaîne grasse; on peut citer à titre d'exemple :
   - les produits ANTARON V216 ou GANEX V216 (copolymère vinylpyrrolidone / hexadécène) vendu par la société I.S.P.
   - les produits ANTARON V220 ou GANEX V220 (copolymère vinylpyrrolidone / eicosène) vendu par la société I.S.P.
- **(4)** les copolymères de méthacrylates ou d'acrylates d'alkyles en C₁-C₆ et de monomères amphiphites comportant au moins une chaîne grasse tels que par exemple le copolymère acrylate de méthyle/acrylate de stéaryle oxyéthyléné vendu par la société GOLDSCHMIDT sous la dénomination ANTIL 208.
- **(5)** les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle.
- **(6)** les polymères à squelette aminoplaste éther possédant au moins une chaîne grasse, tels que les composés PURE THIX proposés par la société SUD-CHEMIE.
- **(7)** les polyuréthanes polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.

De préférence, les polyéthers polyuréthanes comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.

Les polyéthers polyuréthanes peuvent être multiséquencés en particulier sous forme de tribloc. Les séquences hydrophobes peuvent être à chaque extrémité de la chaîne (par exemple: copolymère tribloc à séquence centrale hydrophile) ou réparties à la fois aux extrémités et dans la chaîne (copolymère multiséquencé par exemple). Ces mêmes polymères peuvent être également en greffons ou en étoile.

Les polyéthers polyuréthanes non-ioniques à chaîne grasse peuvent être des copolymères triblocs dont la séquence hydrophile est une chaîne polyoxyéthylénée comportant de 50 à 1000 groupements oxyéthylénés. Les polyéthers polyuréthanes non-ioniques comportent une liaison uréthanne entre les séquences hydrophiles, d'où l'origine du nom.

Par extension figurent aussi parmi les polyéthers polyuréthanes non-ioniques à chaîne grasse, ceux dont les séquences hydrophiles sont liées aux séquences lipophiles par d'autres liaisons chimiques.

A titre d'exemples de polyéthers polyuréthanes non-ioniques à chaîne grasse utilisables dans l'invention, on peut aussi utiliser aussi le Rhéolate 205 à fonction urée vendu par la société RHEOX ou encore les Rhéolates 208 , 204 ou 212, ainsi que l'Acrysol RM 184, l'Aculyn ou Acrysol 44 et l'Aculyn ou Acrysol 46 de la société ROHM & HAAS [l'ACULYN 46 est un polycondensat de polyéthyléneglycol à 150 ou 180 moles d'oxyde d'éthylène, d'alcool stéarylique et de méthylène bis(4-cyclohexyl-isocyanate) (SMDI), à 15% en poids dans une matrice de maltodextrine (4%) et d'eau (81%); l'ACULYN 44 est un polycondensat de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%)].

On peut également citer le produit ELFACOS T210 à chaîne alkyle en C₁₂₋₁₄ et le produit ELFACOS T212 à chaîne alkyle en C₁₈ de chez AKZO.

Le produit DW 1206B de chez ROHM & HAAS à chaîne alkyle en C₂₀ et à liaison uréthanne, proposé à 20 % en matière sèche dans l'eau, peut aussi être utilisé.

On peut aussi utiliser des solutions ou dispersions de ces polymères notamment dans l'eau ou en milieu hydroalcoolique. A titre d'exemple, de tels polymères on peut citer, le Rhéolate 255, le Rhéolate 278 et le Rhéolate 244 vendus par la société RHEOX. On peut aussi utiliser le produit DW 1206F et le DW 1206J proposés par la société ROHM & HAAS.

Les polyéthers polyuréthanes utilisables selon l'invention sont en particulier ceux décrits dans l'article de G. Fonnum, J. Bakke et Fk. Hansen - Colloid Polym. Sci 271, 380.389 (1993).

Les polymères amphiphiles de type cationique comportant au moins une chaîne grasse utilisés dans la présente invention sont choisis de préférence parmi les dérivés de cellulose quaternisée et les polyacrylates à groupements latéraux aminés non cycliques.

Les dérivés de cellulose quaternisée sont, en particulier,
- les celluloses quaternisées modifiées par des groupements comportant au moins une chaîne grasse, tels que les groupes alkyle, arylalkyle, alkylaryle comportant au moins 8 atomes de carbone, ou des mélanges de ceux-ci,
- les hydroxyéthylcelluloses quaternisées modifiées par des groupements comportant au moins une chaîne grasse, tels que les groupes alkyle, arylalkyle, alkylaryle comportant au moins 8 atomes de carbone, ou des mélanges de ceux-ci.

Les radicaux alkyle portés par les celluloses ou hydroxyéthylcelluloses quaternisées ci-dessus comportent de préférence de 8 à 30 atomes de carbone. Les radicaux aryle désignent de préférence les groupements phényle, benzyle, naphtyle ou anthryle.

On peut indiquer comme exemples d'alkylhydroxyéthyl-celluloses quaternisées à chaînes grasses en C₈-C₃₀, les produits QUATRISOFT LM 200, QUATRISOFT LM-X 529-18-A, QUATRISOFT LM-X 529-18B (alkyle en C₁₂) et QUATRISOFT LM-X 529-8 (alkyle en C₁₈) commercialisés par la société AMERCHOL et les produits CRODACEL QM, CRODACEL QL (alkyle en C₁₂) et CRODACEL QS (alkyle en C18) commercialisés par la société CRODA.

Les polyacrylates à groupements latéraux aminés, quaternisés ou non, possèdent par exemple des groupements hydrophobes du type stéareth 20 (alcool stéarylique polyoxyéthyléné(20)).

Comme exemples de polyacrylates à chaînes latérales aminées, on peut citer les polymères 8781- 121 B ou 9492-103 proposés par la société NATIONAL STARCH.

Dans la composition de teinture d'oxydation selon l'invention, parmi les polymères amphiphiles à chaîne grasse, on préfère utiliser un polymère amphiphile de type non inique ou cationique comportant au moins une chaîne grasse.

Selon l'invention, le ou les polymères amphiphiles comportant au moins une chaîne grasse peuvent représenter environ 0,01 à 10% en poids du poids total de la composition. De préférence, cette quantité varie d'environ 0,1 à 5% en poids.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement.

Selon une forme de mise en oeuvre préférée du procédé de teinture de l'invention, on mélange de préférence, au moment de l'emploi, la composition tinctoriale décrite ci-dessus avec une composition oxydante contenant, dans une milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates, les peracides et les enzymes parmi lesquelles on peut citer les peroxydases, les oxydo-réductases à 2 électrons telles que les uricases et les oxygénases à 4 électrons comme les laccases. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ, et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Enfin l'invention a également pour objet le produit coloré résultant de l'oxydation d'au moins un composé de formule (I) telle que définie ci-dessus en présence d'au moins agent oxydant tel que défini précédemment et éventuellement en présence d'au moins un coupleur et/ou d'au moins une base d'oxydation additionnelle.

Ces produits colorés peuvent notamment se présenter sous la forme de pigments et être utilisés à titre de colorant direct pour la teinture directe des cheveux ou bien encore être incorporés dans des produits cosmétiques tels que par exemple dans des produits de maquillage.

Les exemples qui suivent sont destinés à illustrer l'invention.

### Exemple 1 : (R)1-(4-Amino-phenyl)-pyrrolidin-3-ol , dichlorhydrate (2)

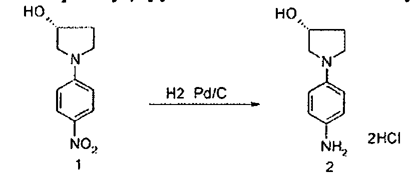

### synthèse du (R)1-(4-Amino-phéyl)-pyrrolidin-3-ol , dichlorhydrate (2):

Dans un hydrogénateur en inox de 200 ml tout équipé, on introduit sous un léger courant d'azote 5 g (24 mmoles) de (R) 1-(4-Nitro-phéhyl)-pyrrolidin-3-ol 1 et 1 g de Palladium sur charbon. On verse ensuite 50ml d'éthanol puis on agite à 1500 tr/mm en purgeant trois fois à l'azote et une fois à l'hydrogène. On introduit ensuite l'hydrogène sous une pression de 5,5 bars. On laisse se développer l'exotherme jusqu'à 55°C. Après une heure et trente minutes, la température est redescendue à 31°C. Le mélange est purgé sous azote puis filtré sur filtre clos sous une pression d'azote de 2 bars. Le filtrat est récupéré dans un mélange glacé de 7 ml d'acide chlorhydrique concentré et de 21 ml d'isopropanol. Le mélange partiellement cristallisé est transféré dans un billon avec 20ml d'isopropanol supplémentaire pour y être ensuite évaporé sous pression réduite. En fin de concentration, le produit cristallise abondamment. On ajoute ensuite 40ml d'isopropanol puis on laisse sous agitation la nuit sous un léger balayage d'azote. Le mélange est alors filtré, essoré, lavé avec 20 ml d'isopropanol et séché sous vide poussé (∼10 mmHg) à la température de 40°C. On obtient ainsi 4,3 g (71%) de (R)1-(4-Amino-phényl)-pyrrolidin-3-ol , dichlorhydrate (2). Les spectres 1 H-RMN et de masse sont conformes aux spectres du produit (3).

| | % C | % H | % N | %O | % Cl |
|---|---|---|---|---|---|
| Théorie | 47.82 | 6.82 | 11.15 | 6.37 | 28.23 |
| Trouvé | 47.27 | 6.40 | 11.06 | 6.99 | 27.95 |

### Exemple 2 : Synthèse du chlorhydrate de 1-(4-aminophényl)-pyrrolidin-3-ol

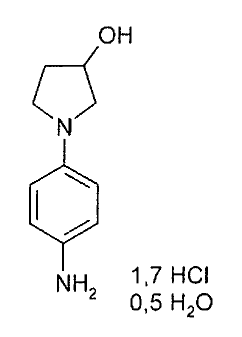

### Etape n°1 : Préparation du 1-(4-nitiophényl)-pyrrolidin-3-ol

Dans un tricol, on a introduit 2 g de 1-fluoro-4-nitrobenzène (0.0155 mol), 1,3 g d'hydrogénocarbonate de sodium (0.0155 mol) et 15 ml d'un mélange dioxanne/eau (8/2). A ce mélange, on a ajouté rapidement 1,35 g de 3-pyrrolidinol (0.0155 mol) racémique. Le mélange hétérogène a été chauffé au reflux (87°C) pendant 10 heures. On a ensuite versé le mélange réactionnel dans de l'eau glacée ; on a obtenu un précipité jaune que l'on a filtré et rincé à l'eau. Après séchage sous vide en présence de P₂O₅, 2,95 g d'un solide jaune ont été obtenus (rendement 97%).

L'analyse RMN ¹H (DMSO d₆, 200 MHz, ppm) conforme au produit attendu était la suivante :
8,04 (d, J = 9 Hz, 2H) ; 6,58 (d, J = 9 Hz, 2H) ; 5,06 (d, J =3,6 Hz, 1H) ; 4,41 (m, 1H) ; 3,45 (m, 3H) ; 3,20 (m, 1H) ; 2,04 (m, 2H).

L'analyse élémentaire était la suivante :

| % | C | H | N | O |
|---|---|---|---|---|
| calculé | 57,89 | 5,81 | 13,45 | 23,05 |
| trouvé | 57,17 | 5,72 | 13,23 | 23,28 |

### Etape n°2 : Préparation du chlorhydrate de 1-(4-aminophényl)-pyrrolidin-3-ol

Le 1-(4-nitrophényl)-pyrrolidin-3-ol obtenu ci-dessus à l'étape précédente (2 g, 9,605 mmol) a été mis en suspension dans 40 ml d'éthanol absolu, 8 ml de cyclohexène et en présence de 1 g de palladium sur charbon humide. On a ensuite fait buller quelques minutes de l'argon dans le milieu réactionnel puis chauffé au reflux pendant 4 heures. Le mélange réactionnel a été filtré dans une fiole à vide contenant 100 ml d'éther diisopropylique, 50 in d'isopropanol et 4 équivalents d'éthanol chlorhydrique (6.2 ml) refroidit à -5°C. Filtration du précipité obtenu rapidement et séchage à l'étuve sous vide à 30 °C. On a obtenu 1,22 g d'un solide légèrement rose avec un rendement de 59% dont le point de fusion est supérieur à 200 °C.

L'analyse RMN ¹H (MeOH d₄, 200 MHz, ppm) conforme au produit attendu était la suivante :
7,26 (d, J = 9 Hz, 2H) ; 7,08 (d, J = 9 Hz, 2H) ; 4,48 (m, 1H) ; 3,54 (m, 3H) ; 3,30 (m, 1H) ; 2,09 (m, 2H).

L'analyse élémentaire était la suivante :

| % | C | H | N | O | Cl |
|---|---|---|---|---|---|
| calculé | 48,18 | 6,70 | 11,24 | 9,64 | 24,23 |
| trouvé | 48,54 | 6,62 | 11,16 | 9 | 24,67 |

Le produit obtenu contient 1,7 molécules d'HCl et 0,5 molécule d'eau.

### Exemple 3: Synthèse du chlorhydrate de 1-(4-amino-2-méthylphényl)-pyrrolidin-3-ol

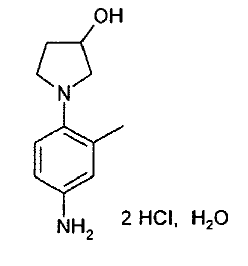

### Etape n°1 : Préparation du 1-(4-nitro-2méthylphényl)-pyrrolidin-3-ol

Dans un tricol, on a introduit 2,4 g de 1-fluoro-2-méthyl-4-nitrobenzène (0.0155 mol), 1,3 g d'hydrogénocarbonate de sodium (0.0155 mol) et 15 ml d'un mélange dioxanne/eau (8/2). A ce mélange, on a ajouté rapidement 1,35 g de 3-pyrrolidinol (0.0155 mol) racémique. Le mélange hétérogène a été chauffé au reflux (87°C) pendant 24 heures. On a ensuite versé le mélange réactionnel dans de l'eau glacée ; on a obtenu un précipité orange que l'on a filtré et rincé à l'eau. Après séchage sous vide en présence de P₂O₅, 3,19 g d'un solide orange ont été obtenus (rendement 93%).

L'analyse RMN ¹H (DMSO d₆, 200 MHz, ppm) conforme au produit attendu était la suivante : 7,80 (m, 2H) ; 6,58 (m, 1H) ; 4,91 (d, J = 3,3 Hz, 1H) ; 4,25 (m, 1H) ; 3,60 (m, 2H) ; 3,41 (m, 1H) ; 3,18 ppm (m, 1H) ; 2,34 (s, 3H) ; 1,82 (m, 2H)

L'analyse élémentaire était la suivante :

| % | C | H | N | O |
|---|---|---|---|---|
| calculé | 59,45 | 6,35 | 12,60 | 21,60 |
| trouvé | 58,91 | 6,40 | 12,20 | 21,42 |

### Etape n°2 : Préparation du chlorhydrate de 1-(4-amino-2-méthylphényl)-pyrrolidin-3-ol

Le 1-(4-nitro-2-méthylphényl)-pyrrolidin-3-ol obtenu ci-dessus à l'étape précédente (2 g, 9,00 mmol) a été mis en suspension dans 40 ml d'éthanol absolu, 8 ml de cyclohexène et en présence de 1 g de palladium sur charbon humide. On a ensuite fait buller quelques minutes de l'argon dans le milieu réactionnel puis chauffé au reflux pendant 4 heures. Le mélange réactionnel a été filtré dans une fiole à vide contenant 100 ml d'éther diisopropylique, 50 ml d'isopropanol et 4 équivalents d'éthanol chlorhydrique (6.2 ml) refroidit à -5°C. Filtration du précipité obtenu rapidement et séchage à l'étuve sous vide à 30 °C. On a obtenu 1,20 g d'un solide légèrement rose avec un rendement de 58%.

L'analyse RMN ¹H (MeOH d₄, 200 MHz, ppm) conforme au produit attendu était la suivante :
7,79 (d, J = 9 Hz, 1H) ; 7,28 (m, 2H) ; 4,58 (m, 1H) ; 3,94 (m, 2H) ; 3,53 (m, 1H) ; 3,13 (m, 1H) ; 2,48 (s, 3H) ; 2,20 (m, 2H).

L'analyse élémentaire était la suivante :

| % | C | H | N | O | Cl |
|---|---|---|---|---|---|
| calculé | 46,80 | 7,09 | 9,93 | 11,34 | 24,82 |
| trouvé | 46,58 | 7,31 | 9,39 | 11,26 | 23,34 |

Le produit obtenu contient 2 molécules d'HCl et 1 molécule d'eau.

### Exemple 4: Synthèse du chlorhydrate de 1-(4-amino-3-méthylphényl)-pyrrolidin-3-ol

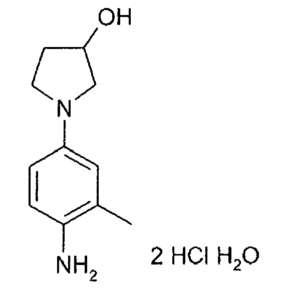

### Etape n°1 : Préparation du 1-(4-nitro-3-méthylphényl)-pyrrolidin-3-ol

Dans un tricol, on a introduit 1,90 ml de 1-fluoro-3-méthyl-4-nitrobenzène (0.0155 mol), 1,3 g d'hydrogénocarbonate de sodium (0.0155 mol) et 15 ml d'un mélange dioxanne/eau (8/2). A ce mélange, on a ajouté rapidement 1,35 g de 3-pyrrolidinol (0.0155 mol) racémique. Le mélange hétérogène a été chauffé au reflux (87°C) pendant 24 heures. On a ensuite versé le mélange réactionnel dans de l'eau glacée ; on a obtenu un précipité jaune que l'on a filtré et rincé à l'eau. Après séchage sous vide en présence de P₂O₅, 2,7 g d'un solide jaune ont été obtenus (rendement 78%).

L'analyse RMN ¹H (DMSO d₆, 200 MHz, ppm) conforme au produit attendu était la suivante : 8.03 (d, J = 9Hz, 1H) ; 6,52 (m, 2H) ; 5,08 (d, J = 3.2, 1H) ; 4,44 (m, 1H) ; 3,50 (m, 3H) ; 3,25 (m, 1H) ; 2,58 (s, 3H) ; 2,05 (m, 2H).

L'analyse élémentaire était la suivante :

| % | C | H | N | O |
|---|---|---|---|---|
| calculé | 59,45 | 6,35 | 12,60 | 21,60 |
| trouvé | 58,82 | 6,48 | 12,16 | 21,50 |

### Etape n°2 : Préparation du chlorhydrate de 1-(4-amino-3-méthylphényl)-pyrrolidin-3-ol

Le 1-(4-nitro-3-méthylphényl)-pyrrolidin-3-ol obtenu ci-dessus à l'étape précédente (2 g, 9,00 mmol) a été mis en suspension dans 40 ml d'éthanol absolu, 8 ml de cyclohexène et en présence de l g de palladium sur charbon humide. On a ensuite fait buller quelques minutes de l'argon dans le milieu réactionnel puis chauffé au reflux pendant 4 heures. Le mélange réactionnel a été filtré dans une fiole à vide contenant 100 ml d'éther diisopropylique, 50 ml d'isopropanol et 4 équivalents d'éthanol chlorhydrique (6.2 ml) refroidit à -5°C. Filtration du précipité obtenu rapidement et séchage à l'étuve sous vide à 30 °C. On a obtenu 1,93 g d'un solide légèrement rose avec un rendement de 94%.

L'analyse RMN ¹H (MeOH d₄, 200 MHz, ppm) conforme au produit attendu était la suivante :
7,44 (m, 1H) ; 7,33 (m, 2H) ; 4,66 (m, 1H) ; 3,79 (m, 3H) ; 3,52 (m, 1H) ; 2,45 (s, 3H) ; 2,37 (m, 2H)

L'analyse élémentaire était la suivante :
% C H N O Cl calculé 46,80 7,09 9,93 11,36 24,82 trouvé 47,49 6,95 9,66 11,32 24,56

Le produit obtenu contient 2 molécules d'HCl et I molécule d'eau.

### EXEMPLES DE TEINTURE

On a préparé les compositions tinctoriales suivantes conformes à l'invention:

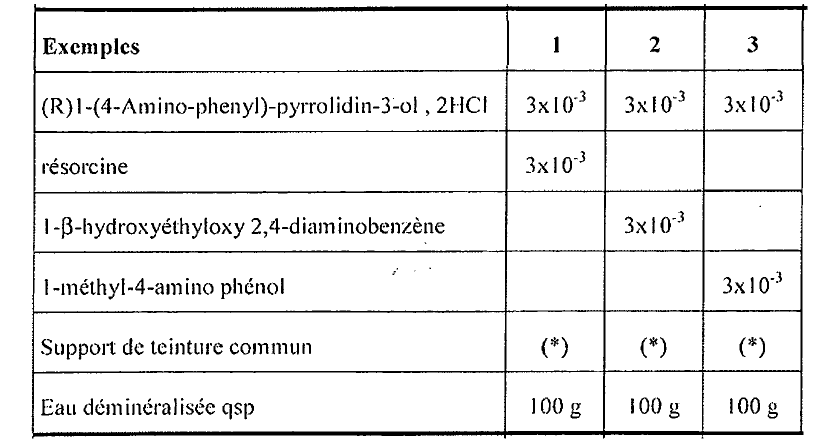

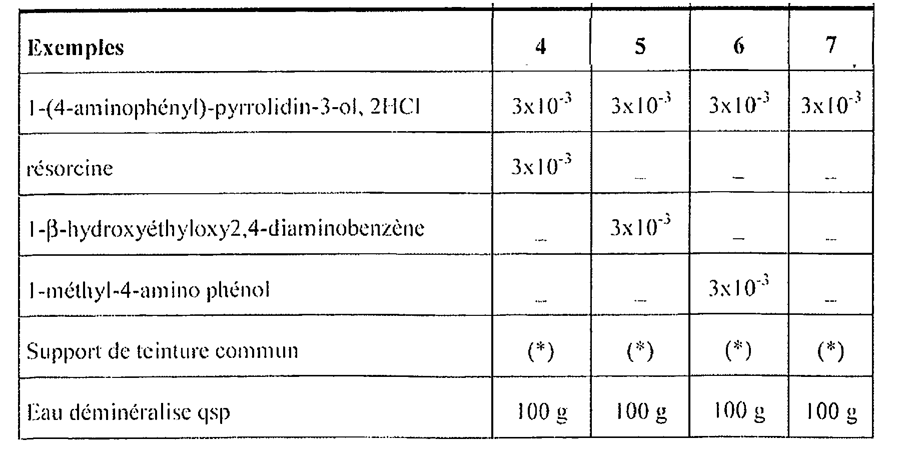

### (*) Support de teinture commun :

- Alcool oléique polyglycérolé à 2 moles de glycérol 4,0 g
- Alcool oléique polyglycérolé à 4 moles de glycérol, à 78 % de
   matières actives (M.A.) 5,69 g M.A.
- Acide oléique 3,0 g
- Amine oléique à 2 moles d'oxyde d'éthylène vendue sous la
   dénomination commerciale ETHOMEEN O12 ® par la société AKZO 7,0 g
- Laurylamino succinamate de diéthylaminopropyle, sel de sodium
   à 55 % de M.A. 3,0 g M.A.
- Alcool oléique 5,0 g
- Diéthanolamide d'acide oléique 12,0 g
- Propylèneglycol 3,5 g
- Alcool éthylique 7,0 g
- Dipropylèneglycol 0,5 g
- Monométhyléther de propylèneglycol 9,0 g
- Métabisulfite de sodium en solution aqueuse, à 35 % de M.A. 0,455 g M.A.
- Acétate d'ammonium 0,8 g
- Antioxydant, séquestrant q.s.
- Parfum, conservateur q.s.
- Ammoniaque à 20 % de NH₃ 10,2 g

Au moment de l'emploi, on mélange chaque composition tinctoriale avec une quantité égale d'une composition oxydante constituée par une solution d'eau oxygénée à 20 volumes (6 % en poids) et présentant un pH d'environ 3.

Chaque mélange obtenu présente un pH d'environ 9,5. Chaque mélange est appliqué sur des mèches de cheveux gris naturels à 90 % de blancs. Après 30 min de pose, les mèches de cheveux sont rincées, lavées avec un shampooing standard rincées puis séchées.

La couleur des mèches a été évaluée dans le système L* a* b*, au moyen d'un spectrophotomètre CM 2002 MINOLTA ®, (Illuminant D65).

Dans le système L* a* b*, les trois paramètres désignent respectivement l'intensité (L*), la composante chromatique rouge-vert (a*) et la composante chromatique jaune-bleu.(b*).

Selon ce système, plus la valeur de L est élevée, plus la couleur est claire ou peu intense. Inversement, plus la valeur de L est faible, plus la couleur est foncée ou très intense.

| Exemples | **Cheveux blancs naturels** | | |
|---|---|---|---|
| | **L*** | **a*** | **b*** |
| **Ex**. **1** | 28,2 | 4,0 | 0,3 |
| **Ex**. **2** | 22,2 | 2,1 | -13,3 |
| **Ex**. **3** | 20,1 | 3,5 | -8,0 |
| **Ex. 4** | 29,9 | 4,44 | 0,58 |
| **Ex. 5** | 23,6 | 0,7 | -13,8 |
| **Ex**. **6** | 22,9 | 5,8 | -14,2 |
| **Ex**. **7** | 47,6 | 1,0 | 8,45 |

On a préparé les compositions tinctoriales suivantes conformes à l'invention:

| **Exemples** | **8** | **9** | **10** | **11** | **12** | **13** |
|---|---|---|---|---|---|---|
| N-(4-aminophényl)-3-hydroxy-pyrrolidine, 2 HCl (composé de formule (I)) | 3 10⁻³ mole | 3 10⁻³ mole | - | - | - | - |
| N-(4-amino-2-méthyl-phényl)-3-hydroxypyrrolidine, 2 HCl (composé de formule (I)) | - | - | 3 10⁻³ mole | 3 10⁻³ mole | - | - |
| N-(4-amino-3-méthyl-phényl)-3-hydroxypyrrolidine, 2 HCl (composé de formule (I)) | - | - | - | - | 3 10⁻³ mole | 3 10⁻³ mole |
| 2,4-diamino I-(β-hydroxy-éthyloxy) benzène, 2 HCl (coupleur) | 3 10⁻³ mole | - | 3 10⁻³ mole | - | 3 10⁻³ mole | - |
| 1,3-dihydroxy benzène (coupleur) | - | 3 10⁻³ mole | - | 3 10⁻³ mole | - | 3 10⁻³ mole |
| Support de teinture commun | (*) | (*) | (*) | (*) | (*) | (*) |
| Eau déminéralisée qsp | 100 g | 100 g | 100 g | 100 g | 100 g | 100 g |

| | | | | | | |
|---|---|---|---|---|---|---|
| (*) Support de teinture commun : identique à celui décrit précédemment | | | | | | |

Au moment de l'emploi, on mélange chaque composition tinctoriale avec une quantité égale d'une composition oxydante constituée par une solution d'eau oxygénée à 20 volumes (6 % en poids) et présentant un pH d'environ 3.

Chaque mélange obtenu présente un pH d'environ 9,5 et est appliqué sur des mèches de cheveux gris naturels à 90 % de blancs. Après 30 minutes, les mèches de cheveux sont ensuite rincées, lavées avec un shampooing standard puis séchées.

Les mèches de cheveux ont été teintes dans les nuances figurant dans le tableau ci-dessous :

| **EXEMPLE** | **NUANCE SUR CHEVEUX NATURELS** |
|---|---|
| **8** | Bleu |
| **9** | Blond irisé violacé cendré |
| **10** | Bleu clair cendré |
| **11** | Beige cendré très légèrement doré |
| **12** | Bleu légèrement mat |
| **13** | Gris cendré légèrement doré |

## Revendications

1. Composition pour la teinture d'oxydation des fibres kératiniques, et en particulier des fibres kératiniques humaines telles que les cheveux, **caractérisée par le fait qu'**elle comprend, dans un milieu approprié pour la teinture, au moins une base d'oxydation choisie parmi les dérivés de paraphénylènediamine à groupement pyrrolidinyle, suivante la N-(4-aminophényl)-3-hydroxy-pyrrolidine, la N-(4-amino-2-méthylphényl)-3-hydroxypyrrolidine, la N-(4-amino-2-éthylphényl)-3-hydroxypyrrolidine, la N-(4-amino-3-méthyl-phényl)-3-hydroxypyrrolidine, la N-(4-amino-3-éthylphényl)-3-hydroxypyrrolidine, et leurs sels d'addition avec un acide et un ou plusieurs coupleurs choisis parmi les métaphénylènediamines, les méta-aminophénols, les métadiphénols et les coupleurs hétérocycliques.

2. Composition selon la revendication précédente, **caractérisée par le fait que** le ou les dérivés de paraphénylènediamine à groupement pyrrolidinyle de formule (I) et/ou le ou leurs sels d'addition avec un acide représentent de 0,0005 à 12% en poids du poids total de la composition tinctoriale, de préférence de 0,005 à 6%.

3. Composition selon l'une quelconque des revendications précédentes dans laquelle le coupleur additionnel est choisi parmi les coupleurs hétérocycliques, les métadiphénols substitués, les métaphénylenediamines substitués, les naphtols, les naphtols acylés et les métaminophénols de formule III suivante 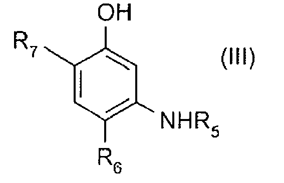 dans laquelle :
- R₅ représente un atome d'hydrogène, un radical alkyle en C₁-C₄, monohydroxyalkyle en C₁-C₄, ou polyhydroxyalkyle en C₂-C₄,
- R₆ représente un atome d'hydrogène, un radical alkyle en C₁-C₄, alcoxy en C₁-C₄, ou un atome d'halogène choisi parmi le chlore, le brome ou le fluor,
- R₇ représente un atome d'hydrogène, un radical alkyle en C₁-C₄, alcoxy en C₁-C₄, monohydroxyalkyle en C₁-C₄, polyhydroxyalkyle en C₂-C₄, monohydroxyalcoxy en C₁-C₄, ou polyhydroxyalcoxy en C₂-C₄.

4. Composition selon la revendication 2 ou 3, **caractérisée par le fait que** les coupleurs sont choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) I-méthoxy benzène, le 1,3-diamino benzène, le 1,3-bis-(2,4-diaminophénoxy) propane, le sésamol, l'α-naphtol, le 2-méthyl-1-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, la 2-amino 3-hydroxypyridine, le 3,6-diméthyl-pyrazolo-[3,2-c]-1,2,4-triazole, le 2,6-diméthyl-pyrazolo-[1,5-b]-1,2,4-triazole, et leurs sels d'addition avec un acide.

5. Composition selon la revendication 2 à 4, **caractérisée par le fait que** le ou les coupleurs représentent de 0,0001 à 10% en poids du poids total de la composition tinctoriale.

6. Composition selon la revendication 4, **caractérisée par le fait que** le ou les coupleurs représentent de 0,005 à 5 % en poids du poids total de la composition tinctoriale.

7. Composition selon l'une quelconque des revendications précédentes, **caractérisée par le fait qu'**elle contient au moins une base d'oxydation additionnelle choisie parmi les paraphénylènediamines différentes des celles définies à la revendication 1, les bis-phénylalkylènediamines, les paraaminophénols, les ortho-aminophénols et les bases hétérocycliques, et leurs sels d'addition avec un acide.

8. Composition selon la revendication 7 dans laquelle la base additionnelle est choisie parmi les bases d'oxydation hétérocycliques, les bases doubles, les paraaminophénols substitués, les orthoaminophénols, les dérivés de paraphénylènediamine de formule (II) suivante, et leurs sels d'addition avec un acide : 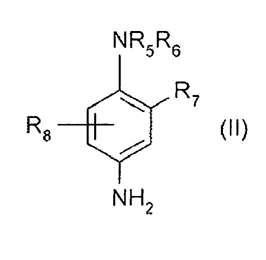 dans laquelle :
- R₅ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un ou plusieurs groupements azotés, phényle ou 4'-aminophényle
- R₆ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄) ou alkyle en C₁-C₄ substitué par un ou plusieurs groupements azotés ;
- R₇ représente un atome d'hydrogène, un atome d'halogène tel qu'un atome de chlore, de brome, d'iode ou de fluor, un radical alkyle en C₁-C₄, monohydroxyalkyle en C₁-C₄, hydroxyalcoxy en C₁-C₄, acétylaminoalcoxy en C₁-C₄, mésylaminoalcoxy en C₁-C₄ ou carbamoylaminoalcoxy en C₁-C₄,
- R₈ représente un atome d'hydrogène, d'halogène ou un radical alkyle en C₁-C₄.

9. Composition selon la revendication 7 ou 8, **caractérisée par le fait que** la ou les bases d'oxydation additionnelles représentent de 0,0005 à 12% en poids du poids total de la composition tinctoriale.

10. Composition selon l'une quelconque des revendications précédentes, **caractérisée par le fait que** les sels d'addition avec un acide sont choisis parmi les chlorhydrates, les bromhydrates, les sulfates, les citrates, les succinates, les tartrates, les lactates, les phosphates et les acétates.

11. Composition selon l'une quelconque des revendications précédentes comprenant au moins un polymère choisi parmi :
- (i)les polymères amphotères,
- (ii)les polymères cationiques contenant des motifs récurrents de structures (II) ou (III) suivantes, et,
- (iii)les polymères différents des précédents qui sont amphiphiles et comportent au moins une chaîne grasse,
- Structure (II) : 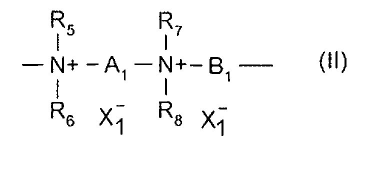 dans laquelle:
R₅, R₆, R₇ et R₈, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₅, R₆, R₇ et R₈, ensemble ou séparément, constituent avec les atomes d'azote auxquels ils sont rattachés des hétérocycles contenant éventuellement un second hétéroatome autre que l'azote ou bien R₅, R₆, R₇ et R₈ représentent un radical alkyle en C₁-C₆ linéaire ou ramifié substitué par un groupement nitrile, ester, acyle, amide ou -CO-O-R₁₃-D ou -CO-NH-R₁₃-D où R₁₃ est un alkylène et D un groupement ammonium quaternaire ;
A₁ et B₁ représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyde, ammonium quaternaire, uréido, amide ou ester.
A₁, R₅ et R₇ peuvent former avec les deux atomes d'azote auxquels ils sont rattachés un cycle pipérazinique ; en outre si A₁ désigne un radical alkylène ou hydroxyalkylène linéaire ou ramifié, saturé ou insaturé, B1 peut également désigner un groupement - (CH₂)n-CO-T-OC-(CH₂)n- dans lequel n est compris entre 1 et 100 et de préférence entre 1 et 50, et T désigne :
a) un reste de glycol de formule : -O-Z-O-, où Z désigne un radical hydrocarboné linéaire ou ramifié ou un groupement répondant à l'une des formules suivantes :
-(CH₂-CH₂-O)x-CH₂-CH₂-
-[CH₂-CH(CH₃)-O]y-CH₂-CH(CH₃)-
où x et y désignent un nombre entier de 1 à 4, représentant un degré de polymérisation défini et unique, ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent
-CH₂-CH₂-S-S-CH₂-CH₂-;
d) un groupement uréylène de formule : -NH-CO-NH-;
X₁ est un anion dérivé d'un acide minéral ou organique et de préférence le chlore ou le brome;
- Structure (III) 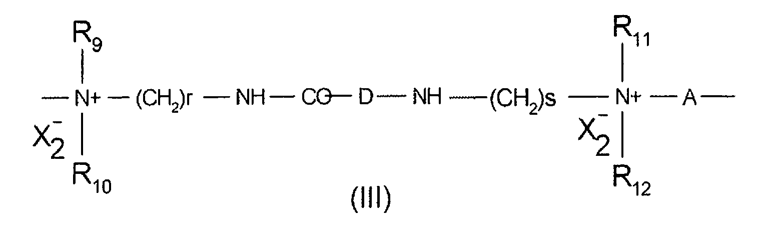 dans laquelle:
R₉, R₁₀, R₁₁ et R₁₂, identiques ou différents, représentent un atome d'hydrogène ou un radical méthyle, éthyle, propyle, β-hydroxyéthyle, β-hydroxypropyle ou -CH₂CH₂ (OCH₂CH₂)pOH, où p est égal à 0 ou à un nombre entier compris entre 1 et 6,
sous réserve que R₉, R₁₀, R₁₁ et R₁₂ ne représentent pas simultanément un atome d'hydrogène,
r et s, identiques ou différents, sont des nombres entiers compris entre 1 et 6,
D est nul ou désigne un groupement -(CH₂)_{q}-CO- dans lequel q est nul ou égal à un nombre entier compris entre 1 et 34,
A désigne le radical d'un dihalogénure ou représente de préférence -CH₂-CH₂-O-CH₂-CH₂-,
X₂⁻ désigne un anion dérivé d'un acide minéral ou organique, de préférence un atome d'halogène.

12. Procédé de teinture d'oxydation des fibres kératiniques, **caractérisé par le fait qu'**on applique sur lesdites fibres au moins une composition tinctoriale telle que définie à l'une quelconque des revendications 1 à 11, et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement.

13. Procédé selon la revendication 12, **caractérisé par le fait que** l'agent oxydant présent dans la composition oxydante est choisi parmi le peroxyde d'hydrogène, le peroxyde d'urée, les bromates de métaux alcalins, les persels, les peracides, et les enzymes.

14. Dispositif à plusieurs compartiments, ou "kit" de teinture à plusieurs compartiments, dont un premier compartiment renferme une composition tinctoriale telle que définie à l'une quelconque des revendications 1 à 11 et un second compartiment renferme une composition oxydante.

## Claims

1. Composition for the oxidation dyeing of keratin fibres, and in particular of human keratin fibres such as the hair, **characterized in that** it comprises, in a medium which is suitable for dyeing, at least one oxidation base chosen from the following para-phenylenediamine derivatives containing a pyrrolidinyl group: N-(4-aminophenyl)-3-hydroxypyrrolidine, N-(4-amino-2-methylphenyl)-3-hydroxypyrrolidine, N-(4-amino-2-ethylphenyl)-3-hydroxypyrrolidine, N-(4-amino-3-methylphenyl)-3-hydroxypyrrolidine and N-(4-amino-3-ethylphenyl)-3-hydroxypyrrolidine, and the addition salts thereof with an acid and one or more couplers chosen from meta-phenylenediamines, meta-aminophenols, meta-diphenols and heterocyclic couplers.

2. Composition according to the preceding claim, **characterized in that** the para-phenylenediamine derivative(s) containing a pyrrolidinyl group of formula (I) and/or the addition salt(s) thereof with an acid represent (s) from 0.0005% to 12% by weight relative to the total weight of the dye composition, and preferably from 0.005% to 6%.

3. Composition according to either of the preceding claims, in which the additional coupler is chosen from heterocyclic couplers, substituted meta-diphenols, substituted meta-phenylenediamines, naphthols, acylated naphthols and the meta-aminophenols of formula III below 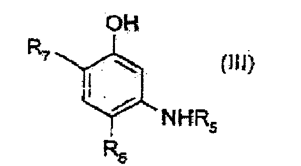 in which:
- R₅ represents a hydrogen atom or a C₁-C₄ alkyl, C₁-C₄ monohydroxyalkyl or C₂-C₄ polyhydroxyalkyl radical,
- R₆ represents a hydrogen atom, a C₁-C₄ alkyl or C₁-C₄ alkoxy radical or a halogen atom chosen from chlorine, bromine and fluorine,
- R₇ represents a hydrogen atom or a C₁-C₄ alkyl, C₁-C₄ alkoxy, C₁-C₄ monohydroxyalkyl, C₂-C₄ polyhydroxyalkyl, C₁-C₄ monohydroxyalkoxy or C₂-C₄ polyhydroxyalkoxy radical.

4. Composition according to Claim 2 or 3, **characterized in that** the couplers are chosen from 2-methyl-5-aminophenol, 5-N-(β-hydroxyethyl)amino-2-methylphenol, 3-aminophenol, 1,3-dihydroxybenzene, 1,3-dihydroxy-2-methylbenzene, 4-chloro-1,3-dihydroxybenzene, 2,4-diamino-1-(β-hydroxyethyloxy)benzene, 2-amino-4-(β-hydroxyethylamino)-1-methoxybenzene, 1,3-diaminobenzene, 1,3-bis(2,4-diaminophenoxy)propane, sesamol, α-naphthol, 2-methyl-1-naphthol, 6-hydroxyindole, 4-hydroxyindole, 4-hydroxy-N-methylindole, 6-hydroxyindoline, 2,6-dihydroxy-4-methylpyridine, 1H-3-methylpyrazol-5-one, 1-phehyl-3-methylpyrazol-5-one, 2-amino-3-hydroxypyridine, 3,6-dimethylpyrazolo[3,2-c]-1,2,4-triazole and 2, 6-dimethylpyrazolo[1,5-b]-1,2,4-triazole, and the addition salts thereof with an acid.

5. Composition according to Claims 2 to 4, **characterized in that** the coupler(s) represent (s) from 0.0001% to 10% by weight relative to the total weight of the dye composition.

6. Composition according to Claim 4, **characterized in that** the coupler(s) represent(s) from 0.005% to 5% by weight relative to the total weight of the dye composition.

7. Composition according to any one of the preceding claims, **characterized in that** it contains at least one additional oxidation base chosen from paraphenylenediamines other than those defined in Claim 1, bis(phenyl)alkylenediamines, para-aminophenols, ortho-aminophenols and heterocyclic bases, and the addition salts thereof with an acid.

8. Composition according to Claim 7, in which the additional base is chosen from heterocyclic oxidation bases, double bases, substituted para-aminophenols, ortho-aminophenols, the para-phenylenediamine derivatives of formula (II) bellow, and the addition salts thereof with an acid: 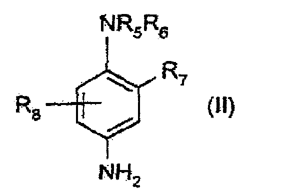 in which:
- R₅ represents a hydrogen atom, a C₁-C₄ alkyl radical, a C₁-C₄ monohydroxyalkyl radical, a C₂-C₄ polyhydroxyalkyl radical, a (C₁-C₄)alkoxy(C₁-C₄) alkyl radical, a C₁-C₄ alkyl radical substituted with one or more nitrogenous groups, a phenyl radical or a 4'-aminophenyl radical;
- R₆ represents a hydrogen atom, a C₁-C₄ alkyl radical, a C₁-C₄ monohydroxyalkyl radical, a C₂-C₄ polyhydroxyalkyl radical, a (C₁-C₄)alkoxy(C₁-C₄)alkyl radical or a C₁-C₄ alkyl radical substituted with one or more nitrogenous groups;
- R₇ represents a hydrogen atom, a halogen atom such as a chlorine, bromine, iodine or fluorine atom, a C₁-C₄ alkyl radical, a C₁-C₄ monohydroxyalkyl radical, a C₁-C₄ hydroxyalkoxy radical, an acetylamioo(C₁-C₄)alkoxy radical, a C₁-C₄ mesylaminoalkoxy radical or a carbamoylamino(C₁-C₄)alkoxy radical,
- R₆ represents a hydrogen or halogen atom or a C₁-C₄ alkyl radical.

9. Composition according to Claim 7 or 8, **characterized in that** the additional oxidation base(s) represent(s) from 0.0005% to 12% by weight relative to the total weight of the dye composition.

10. Composition according to any one of the preceding claims, **characterized in that** the addition sals with an acid are chosen from the hydrochlorides, hydrobromides, sulfates, citrates, succinates, tartrates, lactates, phosphates and acetates.

11. Composition according to any one of the preceding claims comprising at least one polymer chosen *from*:
- (i) amphoteric polymers,
- (ii) cationic polymers containing repeating units of structures (II) or (III) below, and
- (iii) polymers other than the above, which are amphiphilic and comprise at least one fatty chain,
- Structure (II): 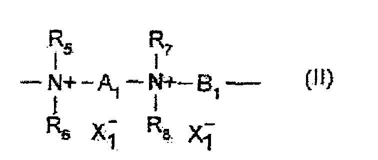 in which:
R₅, R₆, R₇ and R₈, which may be identical or different, represent aliphatic, alicyclic or arylaliphatic radicals containing from 1 to 20 carbon atoms or lower hydroxyalkylaliphatic radicals, or alternatively R₅, R₆, R₇ and R₈, together or separately, constitute, with the nitrogen atoms to which they are attached, heterocycles optionally containing a second heteroatom other than nitrogen, or alternatively R₅, R₆, R₇ and R₈ represent a linear or branched C₁-C₆ alkyl radical substituted with a nitrile, ester, acyl, amide or -CO-O-R₁₃-D or -CO-NH-R₁₃-D group in which R₁₃ is an alkylene and D is a quaternary ammonium group;
A₁ and B₁ represent, polymethylene groups containing from 2 to 20 carbon atoms which may be linear or branched, saturated or unsaturated and which may contain, linked to or intercalated in the main chain, one or more aromatic rings, or one or more oxygen or sulfur atoms or sulfoxide, sulfone, disulfide, amino, alkylamino, hydroxyl, quaternary ammonium, ureido, amide or ester groups.
A₁, R₅ and R₇ may form with the two nitrogen atoms to which they are attached a piperazine ring; in addition, if A₁ denotes a linear or branched, saturated or unsaturated alkylene or hydroxyalkylene radical, B₁ can also denote a group -(CH₂)ₙ-CO-T-OC-(CH₂)ₙ- in which n is between 1 and 100 and preferably between 1 and 50, and
T denotes:
a) a glycol residue of formula: -O-Z-O-, in which Z denotes a linear or branched hydrocarbon-based radical or a group corresponding to one of the following formulae:
-(CH₂-CH₂-O)x-CH₂-CH₂-
-[CH₂-CH(CH₃)-O]y-CH₂-CH(CH₃)-
in which x and y denote an integer from 1 to 4, representing a defined and unique degree of polymerisation, or any number from 1 to 4 representing an average degree of polymerization;
b) a bis-secondary diamine residue such as a piperazine derivative;
c) a bis-primary diamine residue of formula: -NH-Y-NH-, in which Y denotes a linear or branched hydrocarbon-based radical, or alternatively the divalent radical
-CH₂-CH₂-S-S-CH₂-CH₂- ;
d) a ureylene group of formula: -NH-CO-NH-; X₁⁻ is an anion derived from a mineral or organic acid and preferably chlorine or bromine;
- Structure (III) 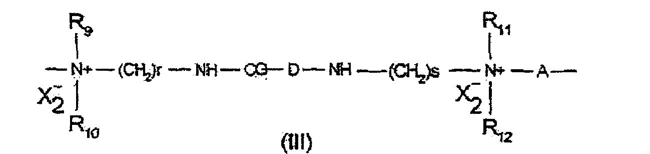 in which:
R₉, R₁₀, R₁₁ and R₁₂, which may be identical or different,
represent a hydrogen atom or a methyl, ethyl, propel, β-hydroxyethyl, β-hydroxypropyl or -CH₂CH₂(OCH₂CH₂)pOH radical, in which p is equal to 0 or to an integer between 1 and 6,
with the proviso that R₉, R₁₀, R₁₁ and R₁₂ do not simultaneously represent a hydrogen atom,
r and s, which may be identical or different, are integers, between 1 and 6,
D is zero or denotes a group -(CH₂)_{q}-CO- in which q is zero or equal to an integer between 1 and 34,
A denotes a dihalide radical or, preferably, represents -CH₂-CH₂-O-CH₂-CH₂-,
X₂⁻ denotes an anion derived from a mineral or organic acid, preferably a halogen atom.

12. Process for the oxidation dyeing of keratin fibres, **characterized in that** at least one dye composition as defined in any one of Claims 1 to 11 is applied to said fibres and **in that** the colour is revealed at acidic, neutral or alkaline pH with the aid of an oxidizing agent which is added just at the time of use to the dye composition, or which is present in an oxidizing composition that is applied simultaneously or sequentially.

13. Process according to Claim 12, **characterized in that** the oxidizing agent present in the oxidizing composition is chosen from hydrogen peroxide, urea peroxide, alkali metal bromates, persalts, peracids and enzymes.

14. Multi-compartment device or multi-compartment dyeing "kit", a first compartment of which contains a dye composition as defined in any one of Claims 1 to 11, and a second compartment of which contains an oxidizing composition.

## Patentansprüche

1. Zusammensetzung zum oxidativen Farben von Keratinfasern und insbesondere menschlichen Keratinfasern, wie Haaren,
**dadurch gekennzeichnet, dass** sie in einem zum Färben geeigneten Medium mindestens eine Oxidationsbase, die unter den folgenden p-Phenylendiaminderivaten mit Pyrrolidinylgruppe: N-(4-Aminophenyl)-3-hydroxypyrrolidin, N-(4-Amino-2-methyl-phenyl)-3-hydroxypyrrolidin, N-(4-Amino-2-ethyl-phenyl)-3-hydroxypyrrolidin, N-(4-Amino-3-methyl-phenyl)-3-hydroxypyrrolidin und N-(4-Amino-3-ethyl-phenyl)-3-hydroxypyrrolidin und deren Additionssalzen mit einer Säure ausgewählt ist, und einen oder mehrere Kuppler enthält, die unter den m-Phenylendiaminen, m-Aminophenolen, m-Dihydroxybenzolen und den heterocyclischen Kupplern ausgewählt sind.

2. Zusammensetzung nach dem vorhergehenden Anspruch,
**dadurch gekennzeichnet, dass** das oder die p-Pherxylendiamin-derivat(e) mit Pyrrolidinylgruppe der Formel (I) und/oder das oder die Additionssalze dieser Verbindungen mit einer Säure 0,0005 bis 12 Gew.-%, des Gesamtgewichts der Farbmittelzusammensetzung und vorzugsweise 0,005 bis 6 Gew.-% ausmachen.

3. Zusammensetzung nach einem der vorhergehenden Ansprüche,
wobei der ergänzende Kuppler unter den heterocyclischen Kupplern, substituierten Dihydroxybenzolen, substituierten m-Phenylendiaminen, Naphtholen, acylierten Naphtholen und m-Aminophenolen der folgenden Formel (III) ausgewählt ist: 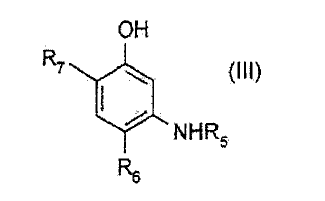 in der Formel:
- R₅ bedeutet ein Wasserstoffatom, eine C₁₋₄-Alkylgruppe, eine C₁₋₄-Monohydroxyalkylgruppe oder eine C₂₋₄-Polyhydroxyalkylgruppe,
- R₆ bedeutet ein Wasserstoffatom, eine C₁₋₄-Alkylgruppe, eine C₁₋₄-Alkoxygruppe oder ein Halogenatom, das unter Chlor, Brom oder Fluor ausgewählt ist,
- R₇ bedeutet ein Wasserstoffatom, eine C₁₋₄-Alkylgruppe, eine C₁₋₄-Alkoxygruppe, eine C₁₋₄-Monohydroxyalkylgruppe oder eine C₂₋₄-Polyhydroxyalkylgruppe, eine C₁₋₄-Monohydroxyalkoxygruppe oder eine C₂₋₄-Polyhydroxyalkoxygruppe.

4. Zusammensetzung nach Anspruch 2 oder 3, **dadurch gekennzeichnet, dass** die Kuppler unter 2-Methyl-5-aminophenol, 5-N-(β-Hydroxyethyl)amino-2-methylphenol, 3-Aminophenol, 1,3-Dihydroxybenzol, 1,3-Dihydroxy-2-methylbenzol, 4-Chlor-1,3-dihydroxybenzol, 2,4-Diamino-1-(β-hydroxyethyloxy)-benzol, 2-Amino-4-(β-Hydroxyethylamino)-1-methoxy-benzol, 1,3-Diaminobenzol, 1,3-Bis(2,4-diaminophenoxy)-propan, Sesamol, α-Naphthol, 2-Methyl-1-naphthol, 6-hydroxyindol, 4-Hydroxyindol, 4-Hydroxy-N-methylindol, 6-Hydroxyindolin, 2,6-Dihydroxy-4-methylpyridin, 1-H-3-Methylpyrazol-5-on, 1-Phenyl-3-methylpyrazol-5-on, 2-Amino-3-hydroxypyridin, 3,6-Dimethyl-pyrazolo-[3,2-c]-1,2,4-triazol, 2,6-Dimethyl-pyrazolo-[1,5-b]-1,2,4-triazol und deren Additioilssalzen mit einer Säure ausgewählt sind.

5. Zusammensetzung nach Anspruch 2 bis 4, **dadurch gekennzeichnet, dass** der oder die Kuppler 0,0001 bis 10 Gew.-% des Gesamtgewichts der Farbmittelzusammensetzung ausmachen.

6. Zusammensetzung nach Anspruch 4, **dadurch gekennzeichnet, dass** der oder die Kuppler 0,005 bis 5 Gew.-% des Gesamtgewichts der Farbmittelzusammensetzung ausmachen.

7. Zusammensetzug nach einem der vorhergehenden Ansprüche, **dadurch gekennzeichnet, dass** sie mindestens eine ergänzende Oxidationsbase enthält, die unter den para-Phenylendiaminen, die von den in Anspruch 1 definierten Verbindungen verschieden sind, Bisphenylalkylendiaminen, para-Aminophenolen, ortho-Aminophenolen und den heterocyclischen Basen und den Additionssalzen dieser Verbindungen mit einer Säure ausgewählt sind.

8. Zusammensetzung nach Anspruch 7, wobei die ergänzende Oxidationsbase unter den heterocyclischen Oxidationsbasen, Doppelbasen, substituieren p-Aminophenolen, o-Aminophenolen, p-Phenylendiaminderivaten der folgenden Formel (II) und ihren Additionssalzen mit einer Säure ausgewählt sind: 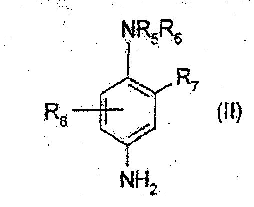 in der Formel bedeuten:
R₅ ein Wasserstoffatom, eine C₁₋₄-Alkylgruppe, eine C₁₋₄- Monohydroxyalkylgruppe, eine C₂₋₄-Polyhydroxyalkylgruppe, eine Alkoxy(C₁₋₄)alkyl(C₁₋₄)gruppe, eine C₁₋₄-Alkylgruppe, die mit einer oder mehreren stickstoffhaltigen Gruppen substituiert ist, eine Phenylgruppe oder eine 4'-Amimophenylgruppe;
R₆ ein Wasserstoffatom, eine C₁₋₄-Alkylgruppe, eine C₁₋₄-Mono- hydroxyalkylgruppe, eine C₂₋₄-Polyhydroxyalkylgruppe, eine Alkoxy(C₁₋₄)alkyl(C₁₋₄)gruppe oder eine C₁₋₄-Alkylgruppe, die mit einer oder mehreren stickstoffhaltigen Gruppen substituiert ist;
R₇ ein Wasserstoffatom, ein Halogenatom, wie Chlor, Brom, Iod oder Fluor, eine C₁₋₄-Alkylgruppe, eine C₁₋₄-Monohydroxyalkyl- gruppe, eine C₁₋₄-Hydroxyalkoxygruppe, eine Acetylaminoalkoxy(C₁₋₄)gruppe; eine Mesylaminoalkoxy(C₁₋ ₄)gruppe oder eine Acetylaminoalkoxy(C₁₋₄)gruppe;;
R₈ ein Wasserstoffatom, ein Halogenatom oder eine C₁₋₄- Alkylgruppe.

9. Zusammensetzung nach Anspruch 7 oder 8, **dadurch gekennzeichnet, dass** die ergänzende(n) Oxidationsbase(n) 0,0005 bis 12 Gew.-% des Gesamtgewichts der Farbmittelzusammensetzung ausmachen.

10. Zusammensetzung nach einem der vorhergehenden Ansprüche, **dadurch gekennzeichnet, dass** die Additionssalze mit einer Säure unter den Hydrochloriden, Hydrobromiden, Sulfaten, Citraten, Succinaten, Tartraten, Lactaten, Phosphaten und Acetaten ausgewählt sind.

11. Zusammensetzung nach einem der vorhergehenden Ansprüche, die mindestens ein Polymer enthält, das ausgewählt ist unter:
(i) amphoteren Polymeren,
(ii) kationischen Polymeren, die Wiederholungseinheiten der folgenden Strukturen (II) oder (II) enthalten, und
(iii) von den vorhergehenden Verbindungen verschiedene Polymere, die amphoter sind und mindestens eine Fettkette enthalten, 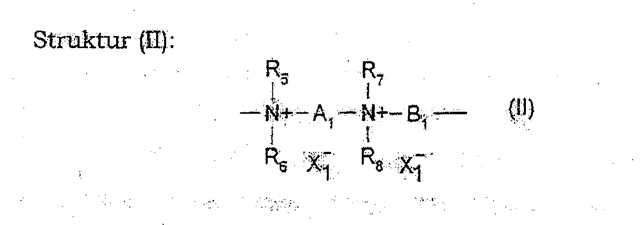 in der Formel:
die Gruppen R₅, R₆, R₇ und R₈, die identisch oder voneinander verschieden sind, bedeuten aliphatische, alicyclische oder arylaliphatische Gruppen mit 1 bis 20 Kohlenstoffatomen oder niedere hydroxyalkylaliphatische Gruppen oder die Gruppen R₅, R₆, R₇ und R₈ bilden gemeinsam oder unabhängig voneinander mit den Stickstoffatomen, an die sie gebunden sind, Heterocyclen, die gegebenenfalls ein zweites Heteroatom enthalten, das von Stickstoff verschieden ist, oder die Gruppen R₅, R₆, R₇ und R₈ bedeuten eine geradkettige oder verzweigte C₁₋₆-Alkylgruppe, die mit einer Nitrilgruppe, Estergruppe, Acylgruppe oder Amidgruppe oder -CO-O-R₁₃-D oder -CO-NH-R₁₃-D substituiert ist, worin R₁₃ eine Alkylengruppe und D eine quartäre Ammoniumgruppe bedeutet;
A₁ und B₁ bedeuten Polymethylengruppen mit 2 bis 20 Kohlenstoffatomen, die geradkettig oder verzweigt, gesättigt oder ungesättigt sein könnten und die an die Hauptkette gebunden oder in der Hauptkette einen oder mehrere aromatische Ringe oder ein oder mehrere Sauerstoffatome oder Schwefelatome oder eine oder mehrere der folgenden Gruppen enthalten können: Sulfoxid, Sulfon, Disulfid, Amino, Alkylamino, Hydroxy, quartäre Ammoniumgruppen, Ureido, Amid oder Ester;
die Gruppen A₁,R₅ und R₇ können mit den beiden Stickstoffatomen, an die sie gebunden sind, einen Piperazinring bilden; wenn A₁ eine geradkettige oder verzweigte, gesättigte oder ungesättigte Alkylen- oder Hydroxyalkylengruppe bedeutet, kann die Gruppe B₁ auch eine Gruppe -(CH₂)ₙ-CO-T-OC-(CH₂)ₙ- sein, wobei n im Bereich von 1 bis 100 und vorzugsweise im Bereich von 1 bis 50 liegt und worin T bedeutet:
a) einen Glycolrest der Formel: -O-Z-O-, worin Z eine geradkettige oder verzweigte Kohlenwasserstoffgruppe oder eine Gruppe bedeutet, die einer der folgenden Formeln entspricht:
-(CH₂-CH₂-O)ₓ-CH₂-CH₂-
-[CH₂-CH(CH₃)-O]_{y}-CH₂-CH(CH₃)-
worin x und y eine ganze Zahl von 1 bis 4 bedeuten und einen wohl definierten und einzigen Polymerisationsgrad darstellen, oder irgendeine Zahl von 1 bis 4 bedeuten und einen mittleren Polymerisationsgrad angeben;
b) einen bis-sekundären Diaminrest, beispielsweise ein Piperazinderivat,
c) einen bis-primären Diaminrest der Formel: -NH-Y-NH-, worin Y eine geradkettige oder verzweigte Kohlenwasserstoffgruppe bedeutet oder auch die zweiwertige Gruppe
-CH₂-CH₂-S-S-CH₂-CH₂-, oder
d) die Ureylengruppe der Formel: -NH-CO-NH-;
X bedeutet ein Anion, das von einer anorganischen ober organischen Säure abgeleitet ist, vorzugsweise Chlorid oder Bromid. 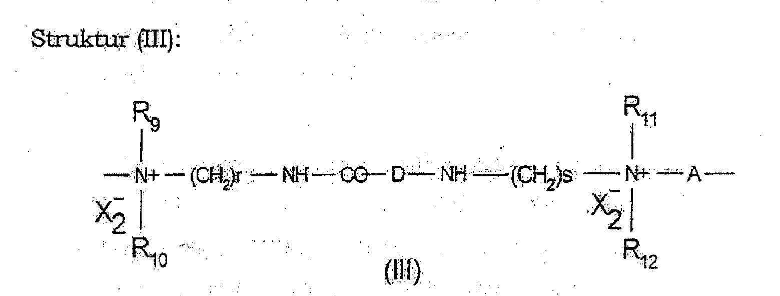 wobei in der Formel:
die Gruppen R₉, R₁₀, R₁₁ und R₁₂, die identisch oder voneinander verschieden sind, bedeuten ein Wasserstoffatom oder Methyl, Ethyl, Propyl, β-Hydroxyethyl, β-Hydroxypropyl oder -CH₂CH₂(OCH₂CH₂)ₚOH, worin p Null oder eine ganze Zahl von 1 bis 6 bedeutet,
mit der Maßgabe, dass die Gruppen R₉, R₁₀, R₁₂ nicht gleichzeitig ein Wasserstoffatom bedeuten,
r und s, die identisch oder voneinander verschieben sind, sind ganze Zahlen im Bereich von 1 bis 6,
D ist nicht vorhanden oder bedeutet eine Gruppe -(CH₂)_{q}-CO-, worin q 0 oder eine ganze Zahl im Bereich von 1 bis 34 bedeutet,
A bedeutet eine Dihalogenidgrupppe oder vorzugsweise -CH₂-CH₂-O-CH₂-CH₂-,
X₂⁻ ist ein Anion, das von einer anorganischen oder organischen Säure abgeleitet ist.

12. Verfahren zum oxidativen Färben von Keratinfasern, **dadurch gekennzeichnet, dass** mindestens eine Farbmittelzusammensetzung, wie sie in einem der Ansprüche 1 bis 11 definiert ist, auf die Fasern aufgebracht und die Farbe bei einem sauren, neutralen oder alkalischen pH-Wert mit einem Oxidationsmittel gebildet wird, das unmittelbar bei der Anwendung zu der Farbmittelzusammensetzung gegeben wird oder das in einer oxidierenden Zusammensetzung vorliegt, die gleichzeitig oder getrennt davon anschließend aufgebracht wird.

13. Verfahren nach Anspruch 12, **dadurch gekennzeichnet, dass** das in der oxidierenden Zusammensetzung enthaltende Oxidationsmittel unter Wasserstoffperoxid, Harnstoffperoxid, Alkalimetallbromaten, Salzen von Persäuren, Persäuren und Enzymen ausgewählt ist.

14. Vorrichtung mit mehreren Abteilungen oder "Kit" zum Färben mit mehreren Abteilungen, wobei eine erste Abteilung eine Farbmittelzusammensetzung, wie sie in einem der Ansprüche 1 bis 11 definiert ist, und die zweite Abteilung eine oxidierende Zusammensetzung enthält.
